(19) Europäisches Patentamt
European Patent Office
Office européen des brevets

(11) **EP 3 793 497 B1**

(12) **EUROPÄISCHE PATENTSCHRIFT**

(45) Veröffentlichungstag und Bekanntmachung des Hinweises auf die Patenterteilung:
**25.09.2024   Patentblatt 2024/39**

(21) Anmeldenummer: **19727595.1**

(22) Anmeldetag: **17.05.2019**

(51) Internationale Patentklassifikation (IPC):
***A61F 13/49*** *(2006.01)*    ***A61F 13/496*** *(2006.01)*
***A61F 13/84*** *(2006.01)*

(52) Gemeinsame Patentklassifikation (CPC):
**A61F 13/49061; A61F 13/496; A61F 13/84;**
A61F 2013/8497

(86) Internationale Anmeldenummer:
**PCT/EP2019/062876**

(87) Internationale Veröffentlichungsnummer:
**WO 2019/219946 (21.11.2019 Gazette 2019/47)**

(54) **INKONTINENZARTIKEL IN HÖSCHENFORM**

INCONTINENCE ARTICLE IN THE FORM OF PANTIES

ARTICLE D'INCONTINENCE SOUS FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2018   DE 102018112121**

(43) Veröffentlichungstag der Anmeldung:
**24.03.2021   Patentblatt 2021/12**

(73) Patentinhaber: **Paul Hartmann AG**
**89522 Heidenheim (DE)**

(72) Erfinder:
  • **BUCH, Tamara**
    **89077 Ulm (DE)**
  • **EILERS, Joerg**
    **89179 Beimerstetten (DE)**
  • **KESSELMEIER, Ruediger**
    **89233 Neu-Ulm (DE)**
  • **BEYRLE, Andreas**
    **89564 Nattheim (DE)**
  • **SCHMIDT, Ann-Cathrin**
    **06667 Weissenfels (DE)**

(74) Vertreter: **DREISS Patentanwälte PartG mbB**
**Friedrichstraße 6**
**70174 Stuttgart (DE)**

(56) Entgegenhaltungen:
**EP-A1- 1 666 012        WO-A1-2006/068541**
**WO-A1-2010/050854    US-A1- 2007 265 591**
**US-A1- 2008 108 967    US-A1- 2016 270 976**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Inkontinenzartikel in Höschenform, also einen Pull-up-Artikel, für die Aufnahme von Körperausscheidungen mit einem vorderen Bauchabschnitt und einem hinteren Rückenabschnitt, die in einer Längsrichtung voneinander beabstandet sind und die zur Bildung eines in Quer- oder Hüftumfangsrichtung durchgehenden Bauch- und Rückenbandes mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in der Längsrichtung zwischen Bauchabschnitt und Rückenabschnitt erstreckt, wobei der Schrittabschnitt, der Bauchabschnitt und der Rückenabschnitt gemeinsam jeweilige Beinöffnungen des Inkontinenzartikels begrenzen, wobei der Bauchabschnitt und der Rückenabschnitt in Quer- oder Hüftumfangsrichtung elastisch dehnbar ausgebildet sind, und wobei der Rückenabschnitt und/oder der Bauchabschnitt ein Vliesmaterial umfasst, auf dem ein erstes Kennzeichnungsmittel aufgebracht ist, und das erste Kennzeichnungsmittel ein erstes Kennzeichen aufweist, das durch eine optische vom Vliesmaterial verschiedene visuell wahrnehmbare farbige flächenhaft erstreckte Zone gebildet ist, und das eine erste Information bereitstellt.

Dabei ist es im Stand der Technik bekannt, Kennzeichnungsmittel auf Inkontinenzartikeln anzubringen. So beschreibt beispielsweise die EP 2 841 038 A1 einen Inkontinenzartikel, der im Hüftrandbereich einen Dehnungsindikator aufweist. Dieser dient zum Erkennen der Passform, wobei bevorzugt eine Referenzgrafik vorgesehen ist.

Insbesondere ist es jedoch wünschenswert, bei derartigen Höschenwindelprodukten eine Markierung anzubringen, die die Vorder- oder Rückseite kennzeichnet. Eine derartige Markierung findet sich auch in regulären Unterwäschen in Form zumeist hinten eingenähter Etiketten.

Die EP 1 815 831 A1 betrifft ebenfalls eine Höschenwindel, mit einer von außen sichtbaren Kennzeichnung auf der Vorder- und/oder Rückseite des Inkontinenzprodukts, wobei eine vertikal verlaufende Mittellinie der Kennzeichnung nicht mit der Längsmittelachse des Inkontinenzprodukts zusammenfällt, so dass die Kennzeichnung seitlich versetzt angeordnet ist. Hierdurch soll erreicht werden, dass die Kennzeichnung weniger prominent erfolgt.

Weitere Inkontinenzartikel mit Kennzeichnungsmitteln sind aus US2007/265591 und US2008/108967 bekannt.

Ausgehend von diesem Stand der Technik war es Aufgabe der Erfindung, ein Kennzeichnungsmittel für einen Inkontinenzartikel bereitzustellen, der eine gute Kennzeichnungskraft aufweist, gleichzeitig jedoch hinreichend dezent ist, wobei zwei Informationen bereitgestellt werden können.

Die Erfindung löst diese Aufgabe durch einen Inkontinenzartikel mit den Merkmalen des Anspruchs 1. Dabei beschreitet die Erfindung den Weg, dass das erste Kennzeichnungsmittel ein zweites Kennzeichen umfasst, das eine zweite Information bereitstellt, und das dadurch gebildet ist, dass in dem ersten Kennzeichen Aussparungen der farbigen Zone vorgesehen sind. Auf diese Weise kann ein zweites Kennzeichen vorgesehen werden, ohne eine weitere farbige Zone einbringen zu müssen, die die Gefahr birgt, dass die verschiedenen Zonen nicht sauber getrennt werden können. Vielmehr wird das zweite Kennzeichen durch die visuell wahrnehmbare Beschaffenheit des Vliesmaterials des ersten Bereichs gebildet, das sich visuell wahrnehmbar von der farbigen Zone abhebt. Hierdurch können vergleichsweise einfach und kostengünstig zwei Informationen durch ein Kennzeichnungsmittel bereitgestellt werden, die keinen zusätzlichen Arbeitsschritt, beispielsweise Einnähen eines Etiketts, benötigen, und dadurch, dass das zweite Kennzeichen durch Aussparungen gebildet wird, die innerhalb der farbigen Zone des ersten Kennzeichens angeordnet sind, können damit in gut erkennbarer und doch dezenter Weise verschiedene Informationen bereitgestellt werden. Insbesondere durch das Einschließen innerhalb einer farbigen Zone wird die zweite Information des zweiten Kennzeichens besser lesbar, als wenn etwa eine herkömmliche Bedruckung ohne den umschließenden Bereich des ersten Kennzeichens vorgesehen wäre. Durch den sich vom zweiten Bereich des Vliesmaterials visuell abhebenden ersten Bereich des ersten Kennzeichens wird ein Kontrast geschaffen, der es ermöglicht, eine vergleichsweise klein dimensionierte Ausbildung des zweiten Kennzeichens bezüglich der Wahrnehmbarkeit zu verbessern.

Insbesondere können die Aussparungen inselförmig sein. Unter "inselförmige" Aussparungen sollen dabei insbesondere solche Aussparungen verstanden werden, die von einem hinreichend breiten Rahmen der farbigen Zone umgeben sind, da dies die Erkennbarkeit der zweiten Information verbessert. Ebenfalls ist unter "inselförmigen" Aussparungen auch eine solche zu verstehen, die durch das Zusammenziehen mehrerer für sich stehender alphanummerischer oder ansonsten für den menschlichen Verstand als separat wahrnehmbare Zeichen zu einer Aussparung gebildet wird.

Die Aussparungen, insbesondere "inselförmigen" Aussparungen, weisen insbesondere alphanumerische Zeichen auf, wie u.a. Buchstaben, Ziffern oder Sonderzeichen.

Insbesondere ist vorgesehen, dass die visuell wahrnehmbare Beschaffenheit des Vliesmaterials innerhalb der Aussparungen derjenigen visuell wahrnehmbaren Beschaffenheit des Vliesmaterials des ersten Bereiches identisch ist.

Unter "Visuell unterscheidbar" wird verstanden, dass die Unterscheidung bereits mit bloßem menschlichen Auge zu erkennen ist, gegebenenfalls kann die Unterscheidbarkeit weiter mit optischen Hilfsmitteln unterstützt werden. "Visuell wahrnehmbar" ist gleichbedeutend mit bloßem menschlichen Auge erkennbar.

Der verwendete Begriff "Farbe" bezieht sich auf die visuelle Wahrnehmungseigenschaft von aus dem Lichtspektrum abgeleiteten und als solche vom Menschen über das Auge erkennbare Lichtleistung. Farben sind typischerweise bekannt wie u.a. Schwarz, Rot, Blau, Grün, Gelb und Mischungen davon.

Die farbige Zone hebt sich somit von der grundlegenden Einfärbung, also der bereits vor Aufbringung des ersten Kennzeichnungsmittels vorherrschenden Farbe des Vliesmaterials im ersten Bereich des Bauch- und oder Rückenabschnitt ab.

Die flächenhaft erstreckte farbige Zone kann auf unterschiedliche Weise im zweiten Bereich des Vliesmaterials eingebracht sein. So ist es denkbar, dass die farbige Zone durch eine Beschichtung oder einen Aufdruck und/oder auf anderem Wege ausgebildet wird.

Unter "Beschichtung" oder "Aufdruck" wird hierbei jeglicher auf die Oberfläche des Vliesmaterials aufgebrachter Zusatz verstanden, der zu einer visuellen, gegebenenfalls verbunden mit einer haptischen und/oder technischen Veränderung des Vliesmaterials führt. Die Beschichtung/der Aufdruck kann als haptisch wahrnehmbar erhoben mit einer gewissen Beschichtungsdicke als einseitig aufliegender Auftrag ausgeführt sein, oder zusätzlich oder ausschließlich als ein das Vliesmaterial durchdringender Auftrag vorgesehen sein.

Die im Stand der Technik bekannten Verfahren zum Erhalt einer farbigen Zone umfassen beispielsweise eine Direktbeschichtung mittels Hochdruck, Tiefdruck, Flachdruck, Rotationsdruck oder auch berührungsloses Beschichten wie Besprühen. Farbige Zonen können aber auch durch Einfärbungsprozesse wie Pigmentdruck, Spritzdruck oder Schablonendruck gestaltet werden.

Materialien und oder Komponenten, die einer solchen farbigen Zone zugeordnet sind, können beispielsweise ein Kleber/ Hotmeltauftrag, eine Druckfarbe als Paste oder eine Tinte sein.

Besonders bevorzugt ist dabei, dass die farbige Zone des ersten Kennzeichnungsmittels eine Bedruckung, insbesondere eine farbige Bedruckung ist. Besonders bevorzugt ist dabei eine einfarbige Bedruckung.

Eine Längsmittelachse des Inkontinenzartikels wird dadurch definiert, dass dieser entlang der Längsmittelachse um eine in Längsrichtung verlaufende Achse mittig gefaltet wird. Auf gleiche Weise wird eine Quermittelachse bestimmt, indem der Inkontinenzartikel in seiner Längsrichtung halbiert wird.

Dabei kann insbesondere vorgesehen sein, dass das erste Kennzeichnungsmittel auf einer körperzugewandten Vlieslage des Vliesmaterials angebracht ist, wobei es dabei auf einer körperzugewandten Oberseite oder einer körperabgewandten Oberseite der körperzugewandten Vlieslage aufgebracht ist, wobei eine körperabgewandte Oberseite besonders bevorzugt ist.

Insbesondere bei einem Vliesmaterial des Bauchabschnitts oder Rückenabschnitts, das zwei äußere, also jeweils eine Außenseite des Vliesmaterials bildende Vlieslagen aufweist, ist das erste Kennzeichnungsmittel zwischen den äußeren Vlieslagen und dabei insbesondere auf der körperabgewandten Oberseite der körperzugewandten Vlieslage aufgebracht. Unter der Anordnung körperzugewandt oder körperabgewandt werden dabei die Richtungen im an einen Träger angelegten bestimmungsgemäßen Zustand verstanden.

Hierdurch wird zum einen erreicht, dass kein Abrieb der farbigen Zone erfolgt, als auch Hautfreundlichkeit durch Vermeidung von direktem Hautkontakt beibehalten wird und bei durch die Vlieslagen bestehender hinreichender Sichtbarkeit gleichwohl das erste Kennzeichnungsmittel weiter optisch in den Hintergrund tritt.

Weiter bevorzugt kann vorgesehen sein, dass ein zweites Kennzeichnungsmittel auf dem das erste Kennzeichnungsmittel aufweisenden Bauch- oder Rückenabschnitt gegenüberliegenden Rücken- oder Bauchabschnitt vorgesehen ist. Dabei ist insbesondere das zweite Kennzeichnungsmittel auf dem Vliesmaterial und dabei insbesondere auf einer körperabgewandten Vlieslage des Vliesmaterials, dabei auf einer körperzugewandten Oberseite oder einer körperabgewandten Oberseite einer körperabgewandten Vlieslage aufgebracht, wobei eine körperzugewandte Oberseite besonders bevorzugt ist. Insbesondere bei einem Vliesmaterial des Bauchabschnitts und/oder Rückenabschnitts mit mindestens zwei äußeren Vlieslagen, also jeweils eine Außenseite des Vliesmaterials bildende Vlieslage, so ist das zweite Kennzeichnungsmittel bevorzugt zwischen den äußeren Vlieslagen und dabei insbesondere auf der körperzugewandten Oberseite der körperabgewandten Vlieslage aufgebracht. Durch die Vorsehung von zweiten Kennzeichnungsmitteln können weitere Informationen transportiert werden. Die Vorsehung des ersten und zweiten Kennzeichnungsmittels differenziert auf jeweils nur einem von Bauchabschnitt oder Rückenabschnitt trägt vorteilhaft zur Differenzierung einer Vorder- und Rückenseite des Inkontinenzartikels bei.

Insbesondere trägt die Anordnung des zweiten Kennzeichnungsmittels auf einer körperabgewandten Vlieslage und die bevorzugte Anordnung des ersten Kennzeichnungsmittels auf einer körperzugewandten Vlieslage vorteilhaft zu einer unterschiedlichen Wahrnehmung der beiden Kennzeichnungsmittel bei, wobei das zweite Kennzeichnungsmittel auf der Produktaußenseite visuell wahrnehmbar noch prägnanter erscheint.

Vorzugsweise wird bei Vorsehung eines ersten und eines zweiten Kennzeichnungsmittels dieselbe Farbgebungs-, insbesondere Beschichtungs- oder Bedruckungstechnik eingesetzt, was verfahrenstechnisch kostengünstig umsetzbar ist. Insbesondere bei einer farbigen Beschichtung, insbesondere einer farbigen Bedruckung weist das erste und zweite Kennzeichnungsmittel die gleiche Farbe auf, was vorteilhaft zu einem homogenen Erscheinungsbild des Inkontinenzartikels beiträgt.

Dabei kann vorgesehen sein, dass der Bauchabschnitt und/oder der Rückenabschnitt Elastifizierungsmittel aufweisen, die sich in einem Abstand voneinander und im Wesentlichen in Quer- oder Hüftumfangsrichtung erstrecken und so den Bauchabschnitt und/oder den Rückenabschnitt flächenhaft elastifizieren, oder dass der Bauchabschnitt und/oder der Rückenabschnitt in Quer- oder Hüftumfangsrichtung elastisch dehnbare Flächenmaterialien umfassen, wobei die Elastifizierungsmittel und/oder die elastisch dehnbaren Flächenmaterialien insbesondere zwischen Vlieslagen des Vliesmaterials angeordnet sind. Eine derartige Elastifizierung ist für Inkontinenzartikel in Höschenform vorteilhaft, da, anders als wiederverschließbare Windeln, Inkontinenzartikel in Höschenform über die Elastifizierung an einen Träger angepasst werden. Die Querelastifizierung ist vom Grundsatz her dadurch möglich, dass im Wesentlichen fadenförmige Elastifizierungsmittel eingesetzt werden oder dass streifenförmige Elastifizierungsmittel oder alternativ auch zumindest in Querrichtung elastisch dehnbare Flächenmaterialien eingesetzt werden. In jedem Fall erweist es sich als vorteilhaft, wenn elastisch dehnbare Materialien mit im Wesentlichen undehnbaren Materialien, wie insbesondere Vliesmaterialien, kombiniert sind.

Insbesondere bevorzugt ist das erste und/oder zweite Kennzeichnungsmittel innerhalb eines elastisch dehnbaren Bereiches von Rücken- und/oder Bauchabschnitt angeordnet.

Bei Anordnung des ersten und/oder zweiten Kennzeichnungsmittels in elastisch dehnbaren Bereichen können mit den unterschiedlichen Dehnungszuständen möglicherweise einhergehenden Konfigurationsänderungen der Kennzeichnungsmittel wiederum Informationen vermittelt werden. Insbesondere können für den Anwender unterschiedliche Farbreize wahrnehmbar sein, so beispielsweise kann das erste Kennzeichen in einem relaxierten, ungedehnten Zustand einen intensiveren Farbeindruck vermitteln als im planen, gedehnten Zustand.

Der Inkontinenzartikel in Höschenform ist insbesondere ein Erwachsenen-Inkontinenzartikel. Insbesondere kann der Inkontinenzartikel als Unisex- Inkontinenzartikel aber auch ergänzend oder abgrenzend als Frauen-Inkontinenzartikel oder als Männer-Inkontinenzartikel vorgesehen sein. Die Ausführungsformen des ersten und/oder zweiten Kennzeichen des ersten Kennzeichnungsmittels als auch dessen Anordnung gegebenenfalls auch zusammen mit einem zweiten Kennzeichnungsmittel vermögen zu dieser Differenzierung beizutragen.

Dabei kann über die Gestaltung des ersten Kennzeichnungsmittels neben der ersten und/oder der zweiten Information eine weitere Information dadurch transportiert werden, dass die farbige Zone neben der Information, wo beispielsweise die Rückseite des Inkontinenzartikels sich befindet, sofern die erste Information einer Positionsmarkierung dient, eine weitere Information beinhalten kann, indem beispielsweise über die gezielte Auswahl einer Farbe der farbigen Zone ein Hinweis über eine Verwendung in geschlechtsspezifischer Weise beinhaltet sein kann. So kann beispielsweise ein erstes Kennzeichnungsmittel in einer blauen Farbe auf einen bevorzugten Einsatzbereich für Männer und ein erstes Kennzeichnungsmittel in einer rosa Farbe auf einen bevorzugten Einsatz für Frauen hinweisen. Alternativ oder auch ergänzend kann die Information des ersten Kennzeichens als dritte in dem Kennzeichnungsmittel enthaltene Information auf eine Saugstärke hinweisen.

Weiter kann über die Form des ersten Kennzeichnungsmittels bzw. über die Form des ersten Kennzeichens, wie beispielsweise Ellipse versus Rechteck, eine weitere Information vermittelt werden.

Darüber hinaus ist es insbesondere vorgesehen, dass das erste Kennzeichnungsmittel so angeordnet ist, dass es eine Information über die Orientierung des Inkontinenzartikels liefert. So ist es beispielsweise vorgesehen, im hinteren Bereich des Inkontinenzartikels erste Kennzeichnungsmittel vorzusehen, die die Rückseite eines Inkontinenzartikels kennzeichnen und markieren. Vorzugsweise ist das erste Kennzeichnungsmittel auf dem Rückenabschnitt angeordnet.

Als zweites Kennzeichen können dann durch die Aussparungen weitere Informationen transportiert werden. Durch die Aussparungen greift das zweite Kennzeichen, die visuell wahrnehmbare Beschaffenheit, wie eben die Farbe des Vliesmaterials des ersten Bereichs auf. Hierdurch wird eine unerwünschte durch visuelle Erscheinung oder Auffälligkeit erzeugte Aufmerksamkeit reduziert, da sich mit einer identischen visuell wahrnehmbaren Beschaffenheit des Vliesmaterials in den Aussparungen in Zusammenschau mit dem Vliesmaterial des das erste Kennzeichnungsmittel umgebenden ersten Bereichs ein gewisses homogenes Erscheinungsbild ergibt. Dabei ist es grundsätzlich vorgesehen, dass das erste Kennzeichnungsmittel zwar hinreichend visuell auffällig sein soll, so dass die Information übermittelt werden kann. Diese Informationsübermittlung soll jedoch vorzugsweise nur an einen Benutzer und/oder Träger des Inkontinenzartikels erfolgen, wohingegen für Dritte das Kennzeichnungsmittel möglichst unauffällig bleiben soll.

Dabei ist besonders bevorzugt vorgesehen, dass die farbige Zone ausgehend von einer Randumfangslinie des ersten Kennzeichnungsmittels, das zweite Kennzeichen derart umschließt, dass die Größe einer Fläche A3, welche von der Randumfangslinie des ersten Kennzeichnungsmittels und einer davon innerhalb und in einem konstanten ersten Abstand AB1 verlaufenden imaginären inneren Abstandslinie begrenzt wird, mindestens 20%, insbesondere mindestens 25%, weiter insbesondere mindestens 30% einer Größe der Fläche A1 beträgt, wobei als die Fläche A1 die durch die Randumfangslinie des ersten Kennzeichnungsmittels eingeschriebene Fläche definiert ist. Weiter vorzugsweise beträgt der Flächenanteil des zweiten Kennzeichens an der Fläche A3 höchstens 10%, insbesondere höchstens 7%, weiter insbesondere höchstens 5%, insbesondere höchstens 2%. Die Bestimmung soll hierbei im vollständig gedehnten Zustand des Inkontinenzartikels erfolgen. Die Größen der Flächen werden jeweils in mm$^2$ vermessen.

Hierdurch wird eine gute visuelle Wahrnehmbarkeit des ersten als auch des zweiten Kennzeichens ermöglicht.

An dieser Stelle sei ausgeführt, dass in der vorliegenden Anmeldung jegliche Bemessungsangaben hinsichtlich Quererstreckung, Längserstreckung oder Abstand von Abschnitten oder von an sich beliebigen Komponenten des Artikels in eben ausgebreitetem Zustand der den Artikel bildenden Flachmaterialien bestimmt werden, also gegebenenfalls nach Auftrennung der herstellerseitig angebrachten Seitennähte, so dass der betreffende Artikel in die in den Figuren dargestellte ebene Konfiguration gebracht werden kann, sofern kein anderer Hinweis auf einen davon abweichenden Zustand angeführt ist. Wenn der Artikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies-Folienverbundbasis auf natürliche Weise.

Durch eine derartige Gestaltung einer Fläche A3, die innerhalb der Fläche A1 liegt, wird der visuelle Eindruck einer zumindest dickeren/breiteren Umrandungsfläche erreicht, die sich in der Art eines Randes um das zweite Kennzeichen erstreckt, wodurch die visuelle Sichtbarkeit und Erkennbarkeit verbessert wird, insbesondere auch bei ansonsten nur schwer lesbaren alphanumerischen Zeichen als zweites Kennzeichen.

Um diese visuelle Erkennbarkeit und das visuell wahrnehmbare Abheben von den ersten Bereichen des Vliesmaterials und als solche das erste Kennzeichnungsmerkmal umgebend weiter zu verbessern, kann vorgesehen sein, dass die Größe einer Fläche A4, welche von der Randumfangslinie des ersten Kennzeichnungsmittels und einer davon außerhalb und in einem konstanten zweiten Abstand AB2 verlaufenden imaginären Abstandslinie begrenzt wird, mindestens 50%, insbesondere mindestens 60%, weiter insbesondere mindestens 70% der Größe der Fläche A1 beträgt, wobei als die Fläche A1 die durch die Randumfangslinie des ersten Kennzeichnungsmittels eingeschriebene Fläche definiert ist. Die Größen der Flächen werden jeweils in mm$^2$ vermessen.

Weiter vorzugsweise sind mindestens 90%, insbesondere mindestens 93%, insbesondere mindestens 95% der Fläche A4 frei von weiteren farbigen Zonen wie Beschichtungen und/oder Bedruckungen und/oder Änderungen der dem Vliesmaterial zu eigenen optischen visuellen und/oder haptischen Beschaffenheit. Die Bestimmung soll hierbei im vollständig gedehnten Zustand des Inkontinenzartikels erfolgen.

Insbesondere kann vorgesehen sein, dass zwischen der Randumfangslinie des ersten Kennzeichnungsmittels, also einer Randumfangslinie der visuell wahrnehmbaren farbigen Zone des ersten Kennzeichens, und einer in einem konstanten ersten Abstand AB1 verlaufenden imaginären inneren Abstandslinie, die einen Abstand von mindestens 2 mm, weiter insbesondere 3 mm, weiter insbesondere mindestens 4 mm und weiter insbesondere mindestens 5 mm aufweist, zumindest abschnittsweise, insbesondere vollumfänglich keine Aussparungen vorgesehen sind.

Darüber hinaus kann insbesondere vorgesehen sein, dass zwischen der Randumfangslinie des ersten Kennzeichnungsmittels, also einer Randumfangslinie der farbigen Zone des ersten Kennzeichens, und einer in einem konstanten zweiten Abstand AB2 verlaufenden imaginären äußeren Abstandslinie, die einen Abstand von mindestens 2 mm, weiter insbesondere mindestens 3 mm, weiter insbesondere mindestens 4 mm und weiter insbesondere mindestens 5 mm aufweist, eine innerhalb dieses zweiten Abstands AB2begrenzten Fläche zumindest abschnittsweise, insbesondere vollumfänglich keine farbige Zone, wie z.B. in Form einer Bedruckung vorgesehen ist.

Weiter kann insbesondere vorgesehen sein, dass das erste Kennzeichnungsmittel eine durch eine Randumfangslinie eingeschriebene Fläche A1 überfängt, und das zweite Kennzeichen darin einen Flächenanteil von höchstens 50%, insbesondere höchstens 40%, weiter insbesondere höchstens 30%, weiter insbesondere höchstens 20% und insbesondere wenigstens 8%, insbesondere wenigstens 10% der vom ersten Kennzeichnungsmittel überfangenen Fläche A1 einnimmt.

Hierdurch bleibt die visuelle Sichtbarkeit des ersten Kennzeichens bestehen.

Auch dies dient dazu, die farbige Zone des ersten Kennzeichens hinreichend erkennbar zu gestalten. Die Berechnung der Flächen und deren Anteile sind auf den eben ausgebreiteten Zustand bezogen.

Das erste Kennzeichnungsmittel weist vorzugsweise eine Fläche A1 von 500 - 4000 mm$^2$, insbesondere von 800 - 3700 mm$^2$, weiter insbesondere von 1100 - 3500 mm$^2$ auf, wobei die Fläche A1 in einem planen ausgebreiteten Zustand des Vliesmaterials bzw. des Inkontinenzartikels gemessen ist.

Das erste Kennzeichnungsmittel betrifft bezüglich des ersten Kennzeichens insbesondere zusammenhängende Flächen, und weißt als Randumfangslinie insbesondere geschlossene Linien, die somit Flächen ausbilden, bevorzugt, wie beispielsweise Kreise, Ellipsen, Ovale, oder aber auch polygone Flächen, also Vielecke auf. D.h., es handelt sich bevorzugt um eine geometrische Figur, die durch einen geschlossenen Streckenzug umschlossen ist.

Das erste Kennzeichnungsmittel und damit das erste Kennzeichen kann dabei in der Ausbildung seiner Randumfangslinie eine einfache geometrische Form besitzen, insbesondere in Form einer Ellipse, eines Ovals oder eines Kreises oder eines Rechteckes oder eines Polygons ausgebildet sein.

Insbesondere bevorzugt können die Inkontinenzartikel mit unterscheidbaren, unterschiedlichen Formen hinsichtlich des ersten Kennzeichnungsmittels bzw. des ersten Kennzeichens versehen sein. Aus unterschiedlichen Formen kann eine Differenzierung des Inkontinenzartikels als Unisex-, Männer- und/oder Frauen-Inkontinenzartikel erhalten werden. So kann vorzugsweise ein Rechteck für ein Männerprodukt und eine Ellipse oder ein Oval für ein Frauenprodukt eingesetzt werden.

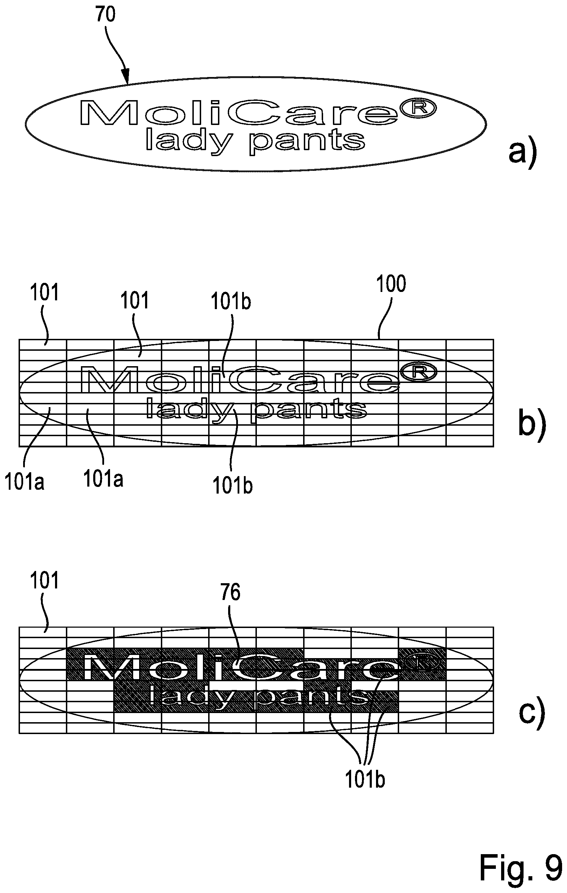

Für die Erkennbarkeit einerseits aber auch die Diskretheit andererseits ist insbesondere eine weitgehend ausgewogene Verteilung des zweiten Kennzeichens innerhalb des ersten Kennzeichens von Vorteil. Die Bestimmung des Verteilungsgrad ist dabei beispielhaft anhand der Figur 9 erläutert.

Es kann dabei bevorzugt vorgesehen sein, dass wenn das erste Kennzeichnungsmittel mit einer Randumfangslinie in seinen jeweils äußersten Punkten von einem imaginären Rechteck tangierend eingeschrieben wird, wobei das Rechteck bezüglich seiner Kanten in Längs- und Querrichtung des Inkontinenzartikels angeordnet ist und dabei sowohl in Längsrichtung als auch in Querrichtung in jeweils 10 gleich große Unterrechtecke unterteilt ist, von einer Anzahl N1 an Unterrechtecken, welche vollständig oder zumindest bereichsweise innerhalb der Randumfangslinie lokalisiert sind und davon einer Anzahl N2 derjenigen Unterrechtecken, die zumindest bereichsweise sowohl das erste Kennzeichen als auch das zweite Kennzeichen aufweisen, ein Verteilungsgrad von N2/N1*100% von mindestens 30%, mindestens 35%, insbesondere mindestens 40%, weiter insbesondere mindestens 45% vorgesehen ist.

Des Weiteren ist es besonders bevorzugt, dass das erste Kennzeichnungsmittel in einem plan ausgebreiteten Zustand des Vliesmaterials und des Inkontinenzartikels eine erste Quererstreckung von 10% bis 25%, insbesondere 12% bis 23% der maximalen Produktquererstreckung aufweist. Hierdurch wird erreicht, dass das erste Kennzeichnungsmittel nicht zu prominent erscheint.

Das erste Kennzeichnungsmittel kann eine zweite Quererstreckung gemessen in einem relaxierten, nicht plan ausgebreiteten Zustand des Inkontinenzartikels aufweisen, wobei die zweite Quererstreckung 4% bis 12%, insbesondere von 5% bis 10% beträgt, dabei bezogen auf die maximale Produktquererstreckung im plan ausgebreiteten Zustand.

Hieraus zeigt sich, dass das erste Kennzeichnungsmittel auf die Vliesmaterialien in einer "überstreckten" Darstellung eingebracht werden kann. Im relaxierten Zustand des Inkontinenzartikels, wie z.B. zum Zeitpunkt der ersten In-Die-Handnahme aus einer Verpackung ist die Dimension des ersten Kennzeichnungsmittels verringert und derart eingestellt, dass Erkennbarkeit und dennoch eine gewisse Diskretion ausgewogen gegeben sind.

Bei einer Einteilung des Inkontinenzartikels in vier gleich große, sich in Längsrichtung erstreckende Teilabschnitte befindet sich vorzugsweise das erste Kennzeichnungsmittel innerhalb der beidseits einer Längsmittelachse des Inkontinenzartikels nächstgelegenen Teilabschnitte. Besonders bevorzugt ist eine im Wesentlichen symmetrisch um die Längsmittelachse angeordnete Vorsehung des ersten Kennzeichnungsmittels. Dabei soll im Wesentlichen symmetrisch noch eine Abweichung von der absoluten Symmetrie von maximal 10%, insbesondere maximal 8% und weiter insbesondere maximal 6% umfassen, ermittelt bezüglich einer Verschiebung einer Längsmittellinie des ersten Kennzeichnungsmittels gegenüber der Längsmittelachse des Inkontinenzartikels. Hierzu wird der maximale Abstand a zwischen der Längsmittellinie des ersten Kennzeichnungsmittels und der Produktseitennaht, der maximale Abstand b zwischen Längsmittelachse des Inkontinenzartikels und der Produktseitennaht ermittelt und daraus wie folgt berechnet als $b-a/b \leq 10\%$, insbesondere $\leq 8\%$, insbesondere $\leq 6\%$.

Des Weiteren kann der Schrittabschnitt ein flüssigkeitsundurchlässiges Backsheetmaterial und ein flüssigkeitsdurchlässiges Topsheetmaterial umfassen, zwischen denen der Absorptionskörper angeordnet ist.

Insbesondere kann es sich bei dem Inkontinenzartikel um einen sogenannten "H-Form"-Artikel handeln, bei dem Schrittabschnitt, Bauchabschnitt und Rückenabschnitt separate Bauteile sind und der Schrittabschnitt an den Bauchabschnitt und an den Rückenabschnitt in einem jeweiligen Überlappungsbereich unlösbar angefügt ist.

Derartige H-Form-Windeln stellen eine für Höschenwindeln besonders günstige Herstellungsvariante dar.

Sofern es sich bei dem Inkontinenzartikel um einen H-Form-Artikel handelt, kann insbesondere vorgesehen sein, dass das erste und/oder zweite Kennzeichnungsmittel außerhalb eines Überlappungsbereichs des Schrittabschnitts mit dem Bauch- und/oder Rückenabschnitt vorgesehen ist.

Hierdurch wird erreicht, dass das Kennzeichnungsmittel nicht in einem Bereich vorgesehen ist, der aufgrund der Materiallagenhäufung eine relativ hohe Opazität aufweist.

Auch bei anderen von der H-Form verschiedenen Ausgestaltungen sind das erste und/oder zweite Kennzeichnungsmittel bevorzugt zumindest außerhalb des Schrittabschnitts angeordnet, insbesondere um auch den Einwirkung der lagenbedingten Opazität zu begegnen und die vollständige Sichtbarkeit des ersten und/oder zweiten Kennzeichnungsmittels bei der ersten In-die-Handnahme nicht durch eine herstellerseitige Faltung im Schrittbereich im Rahmen des Verpackungsprozesses des Inkontinenzartikels zu beeinträchtigen.

Das erste und eventuell auch das zweite Kennzeichnungsmittel ist innerhalb eines Bereichs zwischen einer am Längsende des Absorptionskörpers, insbesondere am Längsende des Schrittabschnitts, angeordneten und in Querrichtung verlaufenden imaginären Linie und einer die Hüftöffnungskante beschreibenden Linie angeordnet, insbesondere in einem Bereich, der in Längsrichtung innerhalb der oberen drei Viertel angeordnet ist.

Es kann dabei insbesondere vorgesehen sein, dass das erste Kennzeichen eine erste Farbe aufweist und das zweite Kennzeichen und/oder das Vliesmaterial in einem an das erste Kennzeichen direkt angrenzenden Bereich eine davon unterscheidbare zweite Farbe aufweist, dabei mit der Farbe jeweils gemessen nach CIEL*a*b*, insbesondere

- a) gemessen jeweils auf einer Produktaußenseite des ersten Kennzeichnungsmittels aufweisenden Bauch- und/oder Rückenabschnitts; und/oder

- b) gemessen jeweils auf einer Produktinnenseite des ersten Kennzeichnungsmittels aufweisenden Bauch- und/oder Rückenabschnitts,

wobei die Messungen a) und b) jeweils in einem ungedehnten Zustand des ersten und zweiten Inkontinenzartikels und/oder jeweils in einem auf 200% gedehnten Zustand des Inkontinenzartikels durchgeführt sind.

Besonders bevorzugt ist dabei, wenn der nach CIEL*a*b* berechnete Farbabstand zwischen der ersten und der zweiten Farbe dargestellt als Delta E ($\Delta$E*) 13 bis 30, insbesondere 13 bis 28 beträgt. Auf diese Weise wird ein Label bereitgestellt, das trotz der farbigen Zone des ersten Kennzeichens dezent ist. Da das zweite Kennzeichen von der Beschaffenheit und/oder Farbe des Vliesmaterials des ersten Bereichs gebildet wird, bestimmt dessen Beschaffenheit/Farbe den Wert des zweiten Kennzeichens. Diese Variante ist insbesondere für Ausführungen des Inkontinenzartikels als Frauen-Inkontinenzartikel oder auch als Unisex-Inkontinenzartikel vorgesehen. Insbesondere bei Ausführungen von Inkontinenzartikeln mit einem weitgehend weißen Vliesmaterial in dem zweiten Bereich, wie es oftmals für Unisex-Inkontinenzartikel typischerweise eingesetzt wird, ist eine größerer Farbabstand, also Delta E ($\Delta$E*) wünschenswert, um damit eine attraktions- und damit aufmerksamkeitssteigernde Ausgestaltung bereitzustellen und damit dem Anwender das Erkennen zu erleichtern.

Alternativ kann bevorzugt vorgesehen sein, dass der nach CIEL*a*b* berechnete Farbabstand zwischen der ersten Farbe des ersten Kennzeichens und der zweiten Farbe des zweiten Kennzeichens dargestellt als Delta E ($\Delta$E*) 5 bis 13, insbesondere 5 bis 10 beträgt. Auf diese Weise liegt ein erstes Kennzeichnungsmittel auf einem Inkontinenzartikel vor, dessen Farbabstände geringer sind, aber noch erkennbar sind. Diese Variante ist insbesondere für Ausführungen des Inkontinenzartikels für Anwender mit Bedarf an geringer optischer Auffälligkeit, so insbesondere als Männer-Inkontinenzartikel vorgesehen.

Bei Betrachtung der Farbeindrücke von Produktinnenseite und Produktaußenseite des ersten Kennzeichnungsmittels aufweisenden Bauch- und/oder Rückenabschnitt im Vergleich, so ist insbesondere vorgesehen, dass ein für den ungedehnten Zustand auf der Produktinnenseite des ersten Kennzeichnungsmittels aufweisenden Bauch- und/oder Rückenabschnitt nach CIEL*a*b* berechneter Farbabstand zwischen dem ersten Kennzeichen und dem zweiten Kennzeichen und/oder dem Vliesmaterial in einem an das erste Kennzeichen direkt angrenzenden Bereich, dargestellt als Delta E ($\Delta$E*)[i/K1-K2/100%] größer ist als ein für den ungedehnten Zustand auf der Produktaußenseite des ersten Kennzeichnungsmittels aufweisenden Bauch- und/oder Rückenabschnitt nach CIEL*a*b* berechneter Farbabstand zwischen dem ersten Kennzeichen und dem zweiten Kennzeichen und/oder dem Vliesmaterial in einem an das erste Kennzeichen direkt angrenzenden Bereich, dargestellt als Delta E ($\Delta$E*)[a/K1-K2/100%].

Dabei beträgt der Unterschied zwischen $\Delta$E*[i/K1-K2/100%] und $\Delta$E*[a/K1-K2/100%] insbesondere wenigstens 0,1, insbesondere wenigstens 0,2, insbesondere wenigstens 1, insbesondere wenigstens 2, weiter insbesondere höchstens 10, weiter insbesondere höchstens 9, weiter insbesondere höchstens 8, weiter insbesondere höchstens 7.

Der zwischen dem ersten und zweiten Kennzeichen auf der Produktinnenseite im ungedehnten Zustand im Vergleich zur Produktaußenseite größere Farbabstand deutet auf einen höheren Kontrast auf der Produktinnenseite hin, was vorteilhaft für die auf der Produktinnenseite gewünscht höhere Attraktion des ersten Kennzeichnungsmittels ist, während auf der Produktaußenseite dennoch eine gewisse Diskretion sichergestellt wird.

Weiter vorzugsweise unterscheiden sich ein im auf 200% gedehnten Zustand auf der Produktinnenseite des ersten Kennzeichnungsmittels aufweisenden Bauch- und/oder Rückenabschnitt nach CIEL*a*b* ermittelter Farbabstand zwischen dem ersten Kennzeichen und dem zweiten Kennzeichen und/oder dem Vliesmaterial in einem an das erste Kennzeichen direkt angrenzenden Bereich, dargestellt als Delta E ($\Delta$E*)[i/K1-K2/200%], von dem im auf 200% gedehnten Zustand auf der Produktaußenseite des ersten Kennzeichnungsmittels aufweisenden Bauch- und/oder Rückenabschnitt nach CIEL*a*b* ermittelter Farbabstand zwischen dem ersten Kennzeichen und dem zweiten Kennzeichen und/oder dem Vliesmaterial in einem an das erste Kennzeichen direkt angrenzenden Bereich, dargestellt als Delta E ($\Delta$E*)[a/K1-K2/200%], um einen Betrag von höchstens 5, weiter insbesondere höchstens 4, weiter insbesondere höchstens 3, insbesondere wenigstens 0,2, insbesondere wenigstens 0,5, insbesondere wenigstens 1. Insbesondere bevorzugt ist dabei der Farbstand $\Delta$E*[i/K1-K2/200%] geringer als der Farbabstand $\Delta$E*[a/K1-K2/200%].

Geringe Differenzen zwischen den Farbabständen auf der Produktinnenseite und Produktaußenseite im jeweils 200% gedehnten Zustand unterstützen vorteilhaft, dass das erste Kennzeichnungsmittel von beiden Seiten diskret erscheint, so auch in einem angezogenen Zustand.

Bei Betrachtung der Farbeindrücke auf der Produktinnenseite des ersten Kennzeichnungsmittels aufweisenden Bauch- und/oder Rückenabschnitt im auf 200% gedehnten und ungedehnten Zustand im Vergleich, so ist insbesondere vorgesehen, dass zwischen dem ersten Kennzeichen und dem zweiten Kennzeichen und/oder dem Vliesmaterial in einem an das erste Kennzeichen direkt angrenzenden Bereich, ein auf einer Produktinnenseite des ersten Kennzeichnungsmittels aufweisender Bauch- und/oder Rückenabschnitt nach CIEL*a*b* berechneter Farbabstand im auf 200% gedehnten Zustand, dargestellt als $\Delta E^*$ [i/K1-K2/200%], geringer ist als ein nach CIEL\*a\*b\* berechneter Farbabstand zwischen dem ersten Kennzeichen und dem zweiten Kennzeichen und/oder dem Vliesmaterial in einem an das erste Kennzeichen direkt angrenzenden Bereich auf einer Produktinnenseite des ersten Kennzeichnungsmittels aufweisendem Bauch- und/oder Rückenabschnitts im ungedehnten Zustand, dargestellt als $\Delta E^*$ [i/K1-K2/100%].

Insbesondere bevorzugt ist eine Ausführungsform mit einer Differenz der Farbabstände von $\Delta E^*$ [i/K1-K2/200%] zu $\Delta E^*$ [1/K1-K2/100%] um einen Wert von mindestens 5, insbesondere mindestens 6, weiter insbesondere mindestens 8, weiter insbesondere höchstens 14, weiter insbesondere höchstens 13, weiter insbesondere höchstens 12.

Bevorzugt kann eine alternative Ausführungsform eine Differenz der Farbabstände von $\Delta E^*$ [i/K1-K2/200%] zu $\Delta E^*$ [1/K1-K2/100%] um einen Wert von mindestens 0,2, insbesondere mindestens 0,5, weiter insbesondere höchstens 5, weiter insbesondere höchstens 3 vorsehen.

Dabei gelten die zuerst genannten Werte insbesondere für ein frauenbestimmtes Produkt oder aber auch für ein Unisex-Produkt, wobei die alternativ als zweites genannten Werte insbesondere für ein für Männer bestimmtes Produkt vorgesehen sein können.

Bei Betrachtung der Farbeindrücke auf der Produktaußenseite des ersten Kennzeichnungsmittels aufweisenden Bauch- und/oder Rückenabschnitt im gedehnten (auf 200%) und ungedehnten Zustand im Vergleich, so kann insbesondere vorgesehen sein, dass ein auf einer Produktaußenseite nach CIEL\*a\*b\* berechneter Farbabstand zwischen dem ersten und dem zweiten Kennzeichen und/oder dem Vliesmaterial in einem an das erste Kennzeichen direkt angrenzenden Bereich bei einer Dehnung von 200%, dargestellt als Delta E ($\Delta E^*$)[a/K1-K2/200%], geringer ist im Vergleich zu einem ungedehnten Zustand, dargestellt als Delta E ($\Delta E^*$)[a/K1-K2/100%], dabei insbesondere eine Differenz um einen Betrag von mindestens 1,5, insbesondere mindestens 2, insbesondere mindestens 2,5 und weiter insbesondere höchstens 7, weiter insbesondere höchstens 6 und weiter insbesondere höchstens 5 aufweisen. Daraus ist im auf 200% gedehnten Zustand, was einen Tragezustand durchaus simuliert, von außen eine erhöhte Diskretion gegeben während gleichzeitig eine gute Kennzeichnungskraft im ungetragenen Zustand auf dem Produkt besteht. Diese Werte sind besonders für ein für Frauen konzipiertes Produkt vorgesehen.

Alternativ kann insbesondere vorgesehen sein, dass ein auf einer Produktaußenseite nach CIEL\*a\*b\* berechneter Farbabstand zwischen ersten und zweiten Kennzeichen bei einer Dehnung von 200%, dargestellt als Delta E ($\Delta E^*$)[a/K1-K2/200%], größer ist im Vergleich zu einem ungedehnten Zustand, dargestellt als Delta E ($\Delta E^*$)[a/K1-K2/100%], wobei der Unterschied, insbesondere bei einem für Männer vorgesehenen Inkontinenzartikel mindestens 1, weiter insbesondere von mindestens 1,5, weiter insbesondere von höchstens 5, weiter insbesondere von höchstens 4,5 und weiter insbesondere von höchstens 4 beträgt. So ist auch hier von außen im ungetragenen Zustand eine größtmögliche Diskretion gegeben ist und ein nicht aufdringliches Erscheinungsbild in beiden Zuständen, dem getragenen und dem nichtgetragenen Zustand, besteht.

Dabei erfolgt die Farbmessung nach CIEL\*a\*b\* wie folgt:

**Farbmessung nach dem CIEL\*a\*b\*-Farbraum und daraus abgeleitete Farbabstände, sogenannte $\Delta E^*$-Werte:**

Die Farbmessung nach L\*a\*b\*, auch genannt CIEL\*a\*b\*-Farbraum, beschreibt und definiert Farben geräteunabhängig. Der CIEL\*a\*b\*-Farbraum ist genormt in der DIN EN ISO 11664-4: 2012-06 Teil 4.

Der CIEL\*a\*b\*-Farbraum basiert im Wesentlichen auf der Darstellung einer Farbe innerhalb eines Koordinatensystems mit drei Achsen. Die Achse L\* repräsentiert die Helligkeit zwischen 0 (schwarz) - 100 (weiß); die a\*-Achse gibt den Grün-oder Rotanteil wider, mit negativen Werten für Grün und positiven Werten für Rot. Die b\*-Achse gibt den Blau- oder Gelb-Anteil wider, dabei mit negativen Werten für Blau und positive Werte für Gelb.

**Prüfgerät**

Für die Farbmessungen wird als Prüfgerät ein Spektralphotometer eingesetzt, wie z.B. das Spectro-guide sphere gloss von BYK-Gardner.

**Probenvorbereitung:**

Die Messprobe bzw. den Artikel auf einer ebenen Fläche flach ausbreiten. Für die Vermessung wird der Artikel in Höschenform gegebenenfalls entlang der Seitennähte aufgetrennt, um für die Vermessung den das erste Kennzeichnungsmittel aufweisenden Bauchabschnitt und/oder Rückenabschnitt vorliegen zu haben. Für die Probengröße ist letztlich die von der Messfeldbegrenzung des Prüfgerätes vorgegebene Messfläche zu beachten.

Bevorzugt werden die Messungen an zwei Zuständen der Messprobe bzw. des Artikels vorgenommen.

- A) Ungedehnt
- B) Auf 200% gedehnt

Für den Messansatz B) wird die Messprobe um 100% gegenüber des relaxierten, ungedehnten Ausgangszustandes gerade und ohne Verwindung in eine der Querrichtung des Inkontinenzartikels entsprechenden Richtung gestreckt, und in diesem Dehnungszustand auf einer dafür geeigneten Unterlage fixiert.

Die Messprobe im ungedehnten Zustand spiegelt den Zustand wider, wie der Anwender das Produkt bei erster In-Die-Handnahme, sowie Entnahme aus der Verpackung, wahrnimmt. Die Messprobe auf 200% gedehnt (d.h. um 100% gedehnt) stellt den Zustand des Inkontinenzartikels im Anwendungszustand bzw. in einem Dehnungszustand, der ohne großen Kraftaufwand einzustellen ist, nach.

Die Messungen A) und B) werden weiter vorzugsweise an der Produktinnenseite und/oder Produktaußenseite des ersten Kennzeichnungsmittels aufweisenden Bauch- und/oder Rückenabschnitt vorgenommen.

Die Produktinnenseite versteht sich dabei als die für den Anwendungszustand körperzugewandte Oberseite und die Produktaußenseite als im Anwendungszustand körperabgewandte Oberseite des Inkontinenzartikels.

Die Messungen werden direkt am Produkt vorgenommen. Um die vom Kennzeichnungsmittel, bzw. vom ersten und zweiten Kennzeichen in der Anwendung vermittelten Farbreize zu analysieren, werden die Farbmessungen an der Produktinnenseite und/oder Produktaußenseite des ersten Kennzeichnungsmittels aufweisenden Bauch- und/oder Rückenabschnitt vorgenommen, unabhängig davon, ob die farbige Zone tatsächlich direkt auf einer die Produktinnenseite oder Produktaußenseite bildenden Oberseite des Vliesmaterials auf/angebracht ist oder nicht. Letzteren Falls wird auf die auf der Produktinnenseite oder Produktaußenseite erkennbaren ersten und zweiten Kennzeichen abgestellt.

Die zu vermessende Oberseite der Messprobe ist dabei dem Prüfgerät und dessen optischen Messfeldbereichen zugewandt angeordnet.

**Kalibrieren:**

Das Prüfgerät ist nach den Herstellervorgaben und dafür vorgesehenen Standards kalibriert.

**Messvorgang:**

Für die hierbei vorzunehmenden Messungen wird eine standardisierte weiße Testkarte als Referenzfläche unterhalb der Messprobe im Bereich des vorgesehenen Messfeldes gelegt, so dass die jeweiligen Farbmessungen stets gegenüber einer Standardfläche durchgeführt werden.

Als Referenzfläche werden die für die Opazitätsmessung vorgesehene "opacity charts" von BYK-Gardner, dabei die Karte #2810 eingesetzt (erhältlich bei BYK-Gardner GmbH, Geretsried, Deutschland).

Diese weiße Referenzflächen-Testkarte weist einen L*a*b*-Wert von

L* von 92,12 $\pm$ 0,79
a* von -0,95 $\pm$ 0,14
b* von 3,69 $\pm$ 0,42 auf,
dabei als Mittelwert $\pm$ 6 sigma (Standardabweichung)

Für den Messvorgang wird der Messkopf des Gerätes senkrecht und ohne zusätzlichen Druck auf die vorbereitete Messprobe und die gewünschte zu messende Oberseite der Messprobe aufgesetzt. Dafür wird, je nach Konstruktion des Prüfgerätes, zuerst mit einer Komponente die eine Messfeldbegrenzung aufweist (sogenannter Probenbeobachter), die für die Messung relevante Stelle der Messprobe auf der Messprobe festgelegt. Diesem Messfeld gegenüber wird dann das Prüfgerät mit seiner Messöffnung aufgesetzt.

Für das Prüfverfahren und den Messvorgang werden am Prüfgerät als Einstellungen vorgenommen:

- Skala: CIEL*a*b*

- Index: $\Delta E^*$

- Lichtart: D65

- Winkel: 10°

- Messöffnung: 11 mm

- im Falle von dazu schmaleren Messbereichen, ist ein Prüfgerät mit einer entsprechend schmäleren Messöffnung zu wählen

Es werden von einer Messprobe n = 6 Messungen vorgenommen.

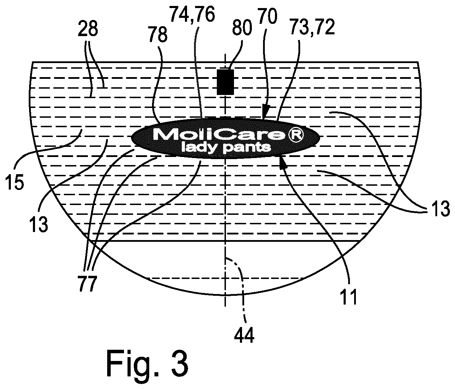

Für die Farbmessung für das erste Kennzeichen ist vorrangig ein jeweiliger Bereich innerhalb der farbigen Zone 73 des ersten Kennzeichens innerhalb des ersten Kennzeichnungsmittels heranzuziehen. Sollte kein genügend großer Bereich zur Verfügung zu stehen, so wird ein Messfeld mit dem größtmöglichen Anteil an erstem Kennzeichen, also an farbiger Zone 73 herangezogen. Für die Farbmessung für das zweite Kennzeichen werden bei ausreichend großen Aussparungen 76 die Farbmessungen innerhalb der Aussparungen 76 vorgenommen. Andernfalls werden die Farbmessungen in einem außerhalb der Randumfangslinie 78 des ersten Kennzeichens direkt angrenzenden Bereich 77 des Vliesmaterials, wie in Figur 3 veranschaulicht, durchgeführt und zwar dort wo sich das Vliesmaterial visuell wahrnehmbar nicht vom Vliesmaterial in den Aussparungen unterscheidet. Dabei wird die Messung an verschiedenen Stellen durchgeführt.

**Auswertung:**

Aus den Messwerten wird der Mittelwert ermittelt.
Die Messwerte und der Mittelwert werden auf eine Dezimale nach dem Komma angegeben.
Die Farbtöne werden als L*, a*, b* Wert angegeben. Der Wert ist dimensionslos.
Für den Vergleich von Farbmessungen an verschiedenen Bereichen bzw. verschiedenen Messproben wird der Farbabstand bei Farbmessungen nach dem CIEL*a*b*-Farbraum als Delta-E ($\Delta E^*$) wie folgt berechnet:

- für die jeweilige Differenz von jeder der drei Achsen des CIEL*a*b*-Farbraums das Quadrat berechnen
- davon die Summe und über diese Summe die Wurzel berechnen:

$$\Delta E^* = [\ (\Delta L^*)^2 + (\Delta a^*)^2 + (\Delta b^*)^2]^{0,5}$$

Der Wert ist dimensionslos.

**Berechnung für die Unterschiede in den L*-Werten:**

Für den Vergleich von Helligkeiten werden von aus den Farbmessungen nach dem CIEL*a*b*-Farbraum erhaltenen Ergebnisse der L*- Koordinate die Unterschiede ($\Delta L^*$) wie folgt berechnet:
Als einfache Differenzmessung zwischen zwei L*-Werten:

$$\Delta L^* = L^*1 - L^*2.$$

Der Wert ist dimensionslos. Als Betrag angegeben wird der Unterschied als solcher beschrieben. Andernfalls kann damit die Richtung der Helligkeitsverschiebung beschrieben werden.
Nach einer weiteren bevorzugten Ausführungsform kann vorgesehen sein, dass der Inkontinenzartikel zum Vertrieb oder im beim Vertrieb dargebotenen Zustand in eine gefaltete Konfiguration gebracht ist. Diese kann derart ausgestaltet sein, dass der das erste Kennzeichnungsmittel aufweisende Bauch- und/oder Rückenabschnitt in der gefalteten Konfiguration des Inkontinenzartikels näher an einer Außenseite der gefalteten Konfiguration angeordnet ist, als der jeweils andere Rücken- oder Bauchabschnitt. So kann eine Faltung beispielsweise dergestalt erfolgen, dass zunächst die beiden Seitennahtbereiche um eine Längsfaltlinie eingefaltet werden, also die Seitenbereiche des Hüftbandes in Richtung auf den Bauchabschnitt. Dann erfolgt eine Querfaltung, wobei beispielsweise ein erstes Kennzeichnungsmittel auf dem Rückenabschnitt vorgesehen sein kann und das erste Kennzeichnungsmittel zwischen den zwei Vlieslagen und hier auf der körperabgewandten Oberseite auf der inneren, d.h. körperzugewandten Lage, angeordnet sein kann.
Besonders bevorzugt ist dabei, wenn das erste Kennzeichnungsmittel auf dem Rückenabschnitt angeordnet ist.
Zur besseren Auffindbarkeit des ersten Kennzeichnungsmittels kann eine weitere Kennzeichnung in Form einer Orientierungsmarke, vorzugsweise zwischen einer Oberkante des ersten Kennzeichnungsmittels und einer Hüftöffnungskante des Inkontinenzartikels, vorgesehen sein. Die Orientierungsmarke dient vorteilhaft zu einem leichteren Auffinden des ersten Kennzeichnungsmittels.
Besonders bevorzugt ist dabei, dass die Orientierungsmarke zentriert zu einer Längsmittelachse des ersten Kennzeichnungsmittels angeordnet ist.
Weiter ist es besonders bevorzugt, wenn die Orientierungsmarke eine dem ersten Kennzeichen identische Farbe aufweist und/oder mit einem identischen die Farbe bildende Mittelgestaltet ist. Alternativ können jedoch auch unterschiedliche Mittel und/oder Verfahren, insbesondere unterschiedliche Beschichtungsarten oder -methoden, sowie unterschiedliche Farben der Beschichtung oder Arten der Beschichtung, z.B. zum einen eine Kleberbeschichtung und zum anderen ein Farbaufdruck, vorgesehen sein.

Die Orientierungsmarke kann vorzugsweise eine kleinere Fläche als die Fläche des ersten Kennzeichnungsmittels aufweisen.

Besonders bevorzugt ist das zweite Kennzeichnungsmittel im Wesentlichen über die gesamte Produktquerstreckung des Bauch- und/oder Rückenabschnitts angeordnet.

Das zweite Kennzeichnungsmittel ist vorzugsweise auf dem Bauchabschnitt angeordnet, und das erste Kennzeichnungsmittel vorzugsweise auf dem Rückenabschnitt. Mit einer solchen Anordnung von erstem und zweitem Kennzeichnungsmittel wird vorteilhaft ein insbesondere für ein Frauenprodukt vorgesehener Inkontinenzartikel bereitgestellt, der herkömmlicher Unterwäsche ähnelt.

Das chassisbildende Vliesmaterial von Bauchabschnitt und/oder Rückenabschnitt des Inkontinenzartikels umfasst vorzugsweise Vliesstoffmaterialien, wie Spinnvliese, Kardenvliese oder Through Air bonded Kardenvliese. Besonders bevorzugt umfasst das Vliesmaterial von Bauchabschnitt und/oder Rückenabschnitt ein Spinnvliesmaterial. Die für Bauchabschnitt und/oder Rückenabschnitt eingesetzten Vliesmaterialien, insbesondere eine jeweilige Vlieslage haben vorteilhafterweise ein Flächengewicht von 10 - 30 $g/m^2$, weiter vorzugsweise von 12 - 25 $g/m^2$, weiter vorzugsweise 12-20$g/m^2$, weiter vorzugsweise von 12 - 18 $g/m^2$.

Als erste und/oder zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt. Die Elastifizierungsmittel haben vorzugsweise eine Stärke von 300-1500 dtex, insbesondere von 500-1200 dtex, weiter insbesondere von 500-900 dtex.

Die Elastifizierungsmittel werden vorzugsweise unter einer Vorspannung von 1,5-6,0, insbesondere von 2,5-5,0 an den chassisbildenden Hüllmaterialien des Bauchabschnitts und/oder Rückenabschnitts (Stretch-bonding Verfahren) fixiert.

Die Vorspannung ist definiert als Dehnungsgrad eines gedehnten Elastifizierungsmittels gegenüber dem ungedehnten/relaxierten Ausgangszustand des Elastifizierungsmittels im Zustand des Aufbringens und Fixierens des Elastifizierungsmittels in der Herstellungsmaschine. Der Dehnungsgrad errechnet sich also als Verhältnis der gedehnten Länge L' (= Ausgangslänge L + $\Delta$L) zur Ausgangslänge L, also L'/L.

Vorzugsweise sind erste Elastifizierungsmittel als im Wesentlichen parallel zur Querrichtung verlaufend eingebracht, vorzugsweise in einem Abstand von 3 - 10 mm, weiter vorzugsweise von 3- 8 mm.

Vorzugsweise können weiter zweite Elastifizierungsmittel, insbesondere in einem schrittseitigen und den Beinöffnungen zugewandten Bereich des Bauchabschnitts und/oder des Rückenabschnitts vorgesehen sein, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.

Weiterhin umfasst die Erfindung eine Anordnung ("Array") umfassend einen ersten Inkontinenzartikel, wie beschrieben, und einen zweiten Inkontinenzartikel in Höschenform, also einen Pull-up-Artikel, für die Aufnahme von Körperausscheidungen mit einem vorderen Bauchabschnitt und einem hinteren Rückenabschnitt, die in einer Längsrichtung voneinander beabstandet sind und die zur Bildung eines in Quer- oder Hüftumfangsrichtung durchgehenden Bauch- und Rückenbandes mit einer in Hüftumfangsrichtung geschlossenen Hüftöffnung an beidseitigen Seitennahtbereichen herstellerseitig miteinander verbunden sind und mit einem einen Absorptionskörper aufweisenden Schrittabschnitt, der sich in der Längsrichtung zwischen Bauchabschnitt und Rückenabschnitt erstreckt, wobei der Schrittabschnitt, der Bauchabschnitt und der Rückenabschnitt gemeinsam jeweilige Beinöffnungen des Inkontinenzartikels begrenzen, wobei der Bauchabschnitt und der Rückenabschnitt in Quer- oder Hüftumfangsrichtung elastisch dehnbar ausgebildet sind, und wobei der Rückenabschnitt und/oder der Bauchabschnitt ein Vliesmaterial mit zumindest einem ersten Bereich und mit einem zweiten Bereich umfasst, wobei in dem ersten Bereich ein erstes Kennzeichnungsmittel vorgesehen ist, und das erste Kennzeichnungsmittel ein erstes Kennzeichen aufweist, das durch eine flächenhaft erstreckte farbige Zone gebildet ist, und das eine erste Information bereitstellt, wobei die farbige Zone visuell unterscheidbar ist von dem zweiten Bereich, wobei das erste Kennzeichnungsmittel des zweiten Inkontinenzartikels ein erstes Kennzeichen umfasst, das eine erste Information bereitstellt, die vom ersten Kennzeichen des ersten Kennzeichnungsmittels des ersten Inkontinenzartikels visuell verschieden ist.

Der erste und der zweite Inkontinenzartikel eines Arrays können die Merkmale des zuvor beschriebenen Inkontinenzartikels aufweisen.

Die Anordnung (Array) ergibt sich dabei durch die sinnfällige Herrichtung der zum Array gehörenden Inkontinenzartikel, insbesondere durch die Beziehung oder das Verhältnis der Artikel zueinander. Diese erfolgt entweder durch Darbietung in einer gemeinsamen Verpackungseinheit und/oder bevorzugt durch die Anbringung von Kennzeichnungen auf den Inkontinenzartikeln und/oder deren Verpackung und/oder die Darbietung in räumlicher oder inhaltlicher Nähe zueinander, die die Zugehörigkeit zu einem Array kenntlich machen. Die den Array bildenden Artikel stammen bevorzugt von ein und demselben Hersteller.

Die einen Array bildenden Inkontinenzartikel weisen bevorzugt dieselbe Produktkennzeichnung auf, wie eben Markennamen und/oder Sub-Markennamen.

EP 3 793 497 B1

Eine Anordnung aus einem ersten Inkontinenzartikel und einem zweiten Inkontinenzartikel wird verstanden als zumindest jeweils einem Vertreter davon und schließt eine Mehrzahl des einen ersten und/oder des einen zweiten Inkontinenzartikels ein oder solche enthaltenden Packungen und Verpackungseinheiten.

Weiter bevorzugt kann vorgesehen sein, dass ein zweites Kennzeichen des zweiten Inkontinenzartikels visuell verschieden ist vom zweiten Kennzeichen des ersten Inkontinenzartikels. Es kann des Weiteren vorgesehen sein, dass das zweite Kennzeichen des zweiten Inkontinenzartikels durch insbesondere inselförmige Aussparungen in der farbigen Zone gebildet ist.

In diesem Fall kann insbesondere vorgesehen sein, dass auch die zweiten Kennzeichen des ersten und zweiten Inkontinenzartikels voneinander verschieden sind. Insbesondere können voneinander verschiedene alphanumerische Zeichen als zweites Kennzeichen vorgesehen sein.

Weiterhin kann vorgesehen sein, dass das erste Kennzeichen des ersten und des zweiten Inkontinenzartikel sich in einer Farbe gemessen nach CIEL*a*b* , vermessen auf der Produktinnenseite und/oder Produktaußenseite unterscheiden, und insbesondere dass ein Farbabstand zwischen den ersten Kennzeichen des ersten und zweiten Inkontinenzartikels, im auf 200% gedehnten Zustand, dargestellt als ΔE* [K1/P1-P2/200%], zwischen 24 bis 34 beträgt. Im ungedehnten Zustand der Inkontinenzartikel beträgt der Farbabstand zwischen den jeweiligen ersten Kennzeichen, dargestellt als ΔE* [K1/P1-P2/100%] vorzugsweise 29 bis 37.

Bei derart hohen Werten der Farbabstände wird hierdurch eine signifikante Unterscheidung der Produkte möglich, insbesondere
bei einem Vergleich des für Frauen vorgesehenen Inkontinenzartikel und/oder auch als Unisex-Produkt vorgesehenen Inkontinenzartikel gegenüber dem für Männer vorgesehenen Inkontinenzartikel.

Weiterhin kann insbesondere vorgesehen sein, dass sich das jeweilige zweite Kennzeichen, vermessen auf der Produktinnenseite und/oder Produktaußenseite des ersten und zweiten Inkontinenzartikel in einer nach CIEL*a*b* gemessenen Farbe voneinander unterscheiden und wobei der Farbabstand insbesondere im auf 200% gedehnten Zustand, dargestellt als ΔE* [K2/P1-P2/200%], 21 bis 27 und im ungedehnten Zustand , dargestellt als ΔE [K2/P1-P2/100%], 29 bis 35 beträgt.

Weiterhin kann vorgesehen sein, dass der erste Inkontinenzartikel zwischen dem ersten Kennzeichen und dem zweiten Kennzeichen und/oder dem Vliesmaterial in einem an das erste Kennzeichen direkt angrenzenden Bereich einen nach CIEL*a*b*-berechneten Farbabstand aufweist, dargestellt als erstes Delta E ΔE*[P1/K1-K2], und der zweite Inkontinenzartikel zwischen dem ersten und dem zweiten Kennzeichen bzw. dem Vliesmaterial in einem an das erste Kennzeichen direkt angrenzenden Bereich einen nach CIEL*a*b*-berechneten Farbabstand aufweist , dargestellt als zweites Delta E ΔE*[P2/K1-K2], wobei die Farbabstände beim ersten Inkontinenzartikel größer sind als beim zweiten Inkontinenzartikel (ΔE*[P1/K1-K2] > ΔE*[P2/K1-K2]) , dabei mit der CIEL*a*b*-Farbmessung

- A) gemessen auf einer Produktaußenseite des ersten Kennzeichnungsmittels aufweisenden Bauch- und/oder Rückenabschnitt und/oder
- B) gemessen jeweils auf einer Produktinnenseite des ersten Kennzeichnungsmittels aufweisenden Bauch- und/oder Rückenabschnitts,
- wobei die Messungen A und B jeweils in einem ungedehnten Zustand des ersten und zweiten Inkontinenzartikels und/oder jeweils in einem gedehnten (auf 200%) Zustand des ersten und zweiten Inkontinenzartikels durchgeführt werden.

Insbesondere ist (ΔE*[P1/K1-K2] größer als ΔE*[P2/K1-K2], insbesondere um den Faktor von wenigstens 1,2, insbesondere wenigstens 1,5, insbesondere höchstens 5, weiter insbesondere höchstens 4,5, weiter insbesondere höchstens 4,0.

Bevorzugt weist der erste Inkontinenzartikel als ein Frauenprodukt und/oder als ein Unisex-Produkt die höheren Farbstände zwischen den Kennzeichen auf.

Weiter kann insbesondere ein Unterschied der Koordinate L* aus dem CIEL*a*b*-Farbraum zwischen dem ersten und dem zweiten Kennzeichen im ersten Inkontinenzartikel größer sein als ein Unterschied der Koordinate L* aus dem CIEL*a*b*-Farbraum zwischen dem ersten und dem zweiten Kennzeichen im zweiten Inkontinenzartikel, wobei die CIEL*a*b*-Farbmessung in den folgenden Messzuständen durchgeführt sein kann:

- A) gemessen auf einer Produktaußenseite des ersten Kennzeichnungsmittels aufweisenden Bauch- und/oder Rückenabschnitt und/oder
- B) gemessen jeweils auf einer Produktinnenseite des ersten Kennzeichnungsmittels aufweisenden Bauch- und/oder Rückenabschnitts,
- wobei die Messungen A und B jeweils in einem ungedehnten Zustand des ersten und zweiten Inkontinenzartikels und/oder jeweils in einem gedehnten (auf 200%) Zustand des ersten und zweiten Inkontinenzartikels durchgeführt werden.

Insbesondere bevorzugt zeigt der erste Inkontinenzartikel als ein vorgesehenes Frauenprodukt einen größeren Differenzbetrag in der Intensität (Helligkeit) als der zweite Inkontinenzartikel als ein vorgesehenes Männerprodukt, sofern das Männerprodukt bereits ein dunkelfarbigeres Vliesmaterial als das Frauenprodukt aufweist. Da sich das Produkt dann schon durch das farbige bzw. dunkelfarbige Vliesmaterial abhebt ist eine auffällige Kennzeichnung im Männerprodukt weniger entscheidend als bei einem Frauenprodukt gegenüber einem Unisexprodukt. Deshalb kann bei einem Männerprodukt die Kennzeichnung dezenter ausgestaltet sein.

Besonders bevorzugt ist dabei, dass der erste Inkontinenzartikel für die Verwendung durch Frauen und/oder als Unisex-Produkt und der zweite Inkontinenzartikel für die Verwendung durch Männer vorgesehen ist, wobei dies durch die erste und/oder die zweite oder eine weitere Information des ersten und/oder zweiten Kennzeichens gekennzeichnet ist.

Weiterhin kann bevorzugt vorgesehen sein, dass ein zweites Kennzeichnungsmittel nur auf dem ersten Inkontinenzartikel vorgesehen ist. Mit einem zweiten Kennzeichnungsmittel ausschließlich auf dem ersten Inkontinenzartikel, der insbesondere für die Verwendung durch Frauen vorgesehen ist, trägt dies weiter vorteilhaft zu einer geschlechtsspezifisch unterschiedlichen Kennzeichnung der Inkontinenzartikel bei. Insbesondere bevorzugt sind die ersten Kennzeichnungsmittel jeweils auf dem Rückenabschnitt vorgesehen und das zweite Kennzeichnungsmittel auf dem Bauchabschnitt.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels.

In der Zeichnung zeigen:

| | |
|---|---|
| Figur 1 | eine Draufsicht auf einen erfindungsgemäßen Inkontinenzartikel, wobei ein Bauchabschnitt, ein Rückenabschnitt und ein die beiden überbrückender und verbindender Schrittabschnitt des Inkontinenzartikels noch nicht zur Bildung einer Höschenform verbunden, sondern in flach gelegtem und eben ausgebreitetem Zustand dargestellt sind; |
| Figur 2 | eine Schnittdarstellung in Längsrichtung durch den Inkontinenzartikel in einer schematischen Darstellung, |
| Figur 3 | einen Ausschnitt aus Figur 1, |
| Figur 4 | eine Gestaltung eines Inkontinenzartikels ähnlich dem in Figur 1 Gezeigten, |
| Figuren 5a und 5b | jeweils eine schematische Ansicht auf eine Produktinnenseite des Rückenabschnitts im ungedehnten und gedehnten Zustand, |
| Figur 6 | in verschiedenen Darstellungen a, b, c und d einen Faltvorgang eines Inkontinenzartikels, |
| Figur 7 | ein Array umfassend einen ersten Inkontinenzartikel 2 sowie einen zweiten Inkontinenzartikel 2', |
| Figur 8 | das Kennzeichnungsmittel 70, mit der Bestimmung eines inneren und eines äußeren Abstands ausgehend von der Randumfangslinie und |
| Figur 9 | eine Bestimmung des Anteils des ersten und zweiten Kennzeichens an der Fläche des ersten Kennzeichnungsmittels 70. |

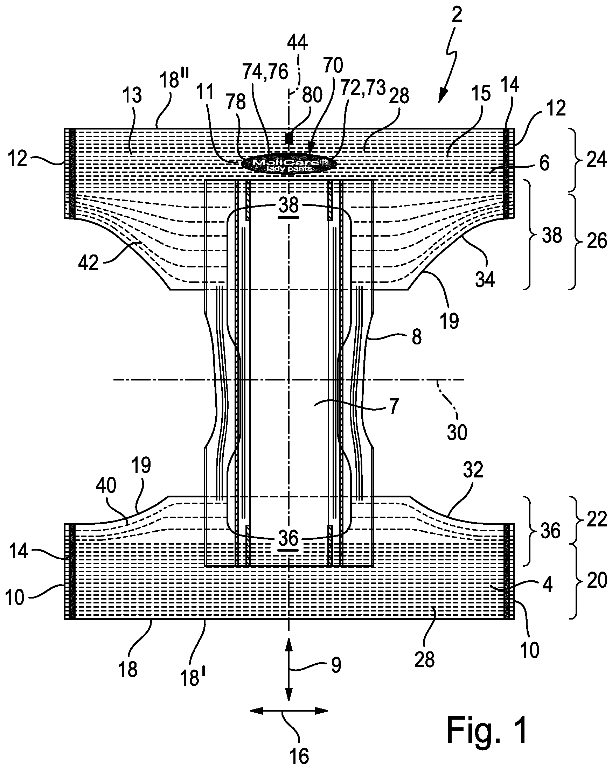

Die Figuren zeigen einen insgesamt mit dem Bezugszeichen 2 bezeichneten Inkontinenzartikel in Höschenform für die Aufnahme fester und flüssiger Körperausscheidungen. Eine besonders bevorzugte Ausführungsform des Inkontinenzartikels 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem Flächenanteil des Bauchschnitts 4 einerseits und des Rückenabschnitts 6 andererseits überlappt und im Überlappungsbereich 36, 38 herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 mit einer vorderen 18' und einer hinteren 18" Hüftöffnungskante und Beinöffnungen 19, durch welche hindurch der Benutzer den Inkontinenzartikel wie ein Höschen anlegt.

Der Bauchabschnitt 4 lässt sich bei der beispielhaften Ausführungsform in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Inkontinenzartikels zulaufende Randkontur 32 bzw. 34. Diese Randkontur 32, 34 ist abschnittsweise bogenförmig und daher zur Begrenzung der Beinöffnungen 19 geeignet. Durch diesen Verlauf des schrittseitigen und den Beinöffnungen zugewandten Bereichs 22 bzw. 26 wird auch ein Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus einer bevorzugten Ausführungsform in den Figuren zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt.

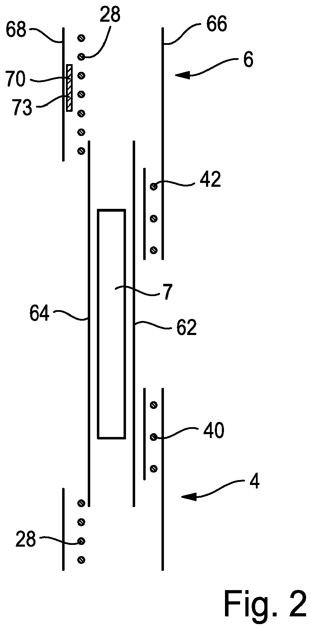

Der Schrittabschnitt 8 umfasst ein flüssigkeitsundurchlässiges Backsheet 62, das insbesondere von einem atmungsaktiven, jedoch flüssigkeitsdichten Folienmaterial gebildet sein kann, und ein, vorzugsweise auf Vliesbasis beruhendes flüssigkeitsdurchlässiges Topsheet 64. Zwischen dem Backsheet 62 und dem Topsheet 64 ist wie aus Figur 2 ersichtlich der Absorptionskörper 7 angeordnet. Im beispielhaft dargestellten Fall bildet das Backsheet 62 und das Topsheet 64 in Längsrichtung 9 einen Überhang über den Absorptionskörper 7.

Darüber hinaus weist der Inkontinenzartikel 2 ein erstes Kennzeichnungsmittel 70 auf. Das Kennzeichnungsmittel ist im vorliegenden Fall im Rückenabschnitt 6 angeordnet. Sowohl der Rückenabschnitt 6 als auch der Bauchabschnitt 4 sind dabei aus einem Vlieslaminat aufgebaut und weisen zwei Vlieslagen 66 und 68 auf, zwischen denen die ersten Elastifizierungsmittel 28 eingebracht sind. Auf der körperabgewandten Oberseite der Vlieslage 68, bei der es sich um die körperzugewandte Vlieslage handelt, ist eine farbige Zone 73 vorgesehen, die das erste Kennzeichnungsmittel 70 bildet. Auf diese Weise wird erreicht, dass kein Abrieb der farbigen Zone erfolgen kann sowie ein direkter Hautkontakt mit der farbigen Zone vermieden wird. Die farbige Zone ist hier in Form eines Farbaufdrucks vorgenommen, wobei, wie in Figur 1 und im vergrößerten Ausschnitt davon in Figur 3 ersehen werden kann, Ausnehmungen 76 in dem ersten Kennzeichnungsmittel vorgesehen sind, die mit einem gewissen Abstand zur Randumfangslinie 78 des ersten Kennzeichnungsmittels angeordnet sind.

Das Vliesmaterial 15 umfasst zumindest einen ersten Bereich 11 und einen zweiten Bereich 13, wobei in dem ersten Bereich 11

das erste Kennzeichnungsmittel 70 vorgesehen ist. Das erste Kennzeichnungsmittel weist dabei ein erstes Kennzeichen 72 auf, das durch die flächenhaft erstreckte farbige Zone 73 gebildet ist und im vorliegenden Fall die Information transportiert, wo die Rückseite des Inkontinenzartikels 2 ist. Darüber hinaus wird durch die Wahl der Farbe eine Information darüber generiert, dass es sich um einen absorbierenden Artikel für einen weiblichen Benutzer handelt. Die Ausnehmungen 76 bilden ein zweites Kennzeichen 74, das eine weitere Information transportiert, nämlich ebenfalls über die Vorsehung von alphanumerischen Zeichen, den Hinweis, dass es sich um ein für Frauen geeignetes Produkt handelt als auch den Hinweis über einen Hersteller bzw. seiner Marke.

Darüber hinaus ist eine Orientierungsmarke 80 vorgesehen, die im Wesentlichen ebenso wie das erste Kennzeichnungsmittel 70 bezüglich der Längsachse 44 spiegelsymmetrisch angeordnet ist und sich in Art eines Strichs in Längsrichtung 9 des Inkontinenzartikels 2 erstreckt. Über die Orientierungsmarke 80 wird das Auffinden des ersten Kennzeichnungsmittels 70 erleichtert.

Dabei bilden die Aussparungen 76 Bereiche in der farbigen Zone 73, in denen insbesondere die visuell wahrnehmbare Beschaffenheit des Vliesmaterials derjenigen visuell wahrnehmbaren Beschaffenheit des Vliesmaterials des ersten Bereichs identisch ist. Auf diese Weise kann eine dezente und doch gut lesbare zweite Information geliefert werden. Insbesondere durch das Einschließen in eine farbige Zone wird die zweite Information des zweiten Kennzeichens besser wahrnehmbar, als wenn eine herkömmliche Ausgestaltung des zweiten Kennzeichens in Form einer herkömmlichen Bedruckung ohne den umschließenden Bereich des ersten Kennzeichens vorgesehen wäre. Durch den sich visuell vom ersten Bereich des Vliesmaterials unterscheidbaren abhebenden zweiten Bereich des ersten Kennzeichens wird ein Kontrast geschaffen, der die vergleichsweise kleinere schlechter erkennbare Information des zweiten Kennzeichens bezüglich der Wahrnehmbarkeit verbessert.

Figur 3 zeigt einen Ausschnitt aus Figur 1.

Dabei ist insbesondere vorgesehen, dass das Kennzeichnungsmittel 70 überstreckt im Vliesmaterial 15 vorgesehen ist, so dass es dann im späteren Anlegezustand, und eben in einem gedehnten Zustand des Elastifizierungsmittel aufweisenden Vliesmaterials sichtbar und bezüglich des alphanumerischen zweiten Kennzeichens lesbar ist.

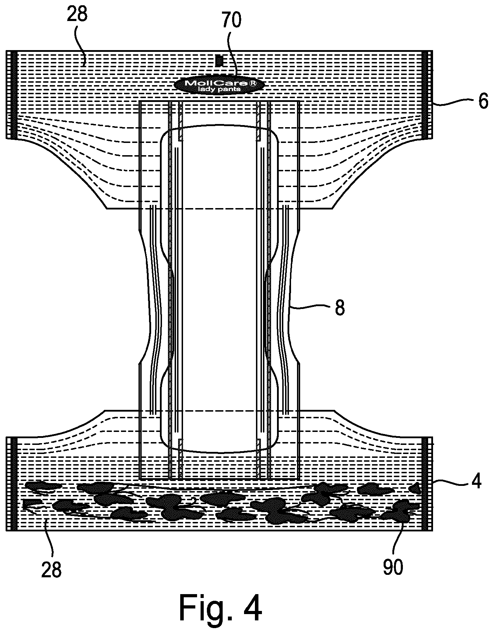

Figur 4 zeigt nun eine Gestaltung eines Inkontinenzartikels ähnlich dem in Figur 1 Gezeigten, wobei hier zusätzlich im Bereich des Bauchabschnitts 4 ein zweites Kennzeichnungsmittel 90 vorgesehen ist, neben der Information für die Vorderseite auch dekorativen Charakter besitzt. Das zweite Kennzeichnungsmittel 90 kann dabei ebenso auf der körperabgewandten Oberseite der Vlieslage, die dem Körper abgewandt ist, des Bauchabschnitts 4 angeordnet sein, sofern es sich hier, wie auch in Figur 2 dargestellt, um einen zweilagigen Aufbau handelt. Insbesondere erstreckt sich das zweite Kennzeichnungsmittel 90 über die gesamte Quererstreckung des Bauchabschnitts und ist ebenso wie das erste Kennzeichnungsmittel 70 im Bereich der ersten Elastifizierungsmittel 28 außerhalb eines Überlappungsbereichs 36, 38 zwischen Schrittabschnitt 8 und Rückenabschnitt 6 sowie Bauchabschnitt 4 vorgesehen.

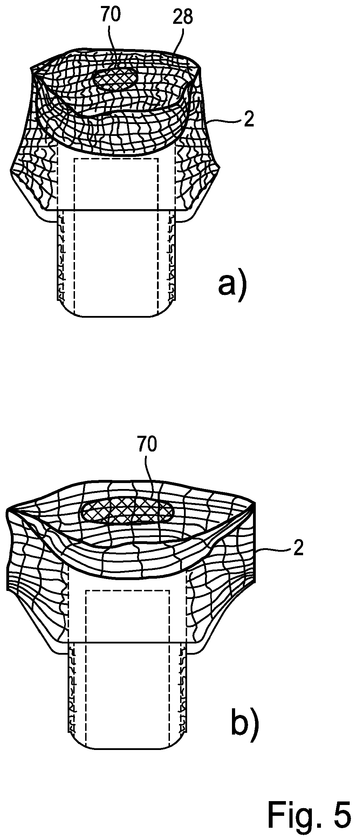

Figur 5 zeigt in den Darstellungen a und b eine Gestaltung, bei der in Figur a im Wesentlichen eine relaxierte Form der Elastifizierungsmittel 28 gezeigt ist, sowie in Figur 5b eine im Gebrauchsfall gedehnte. Dies führt ebenfalls zu einer Dehnung des ersten Kennzeichnungsmittels 70, wodurch das zweite Kennzeichen deutlicher zu erkennen ist. In Figur 5 ist der Inkontinenzartikel 2 schematisch in perspektivischer Darstellung gezeigt.

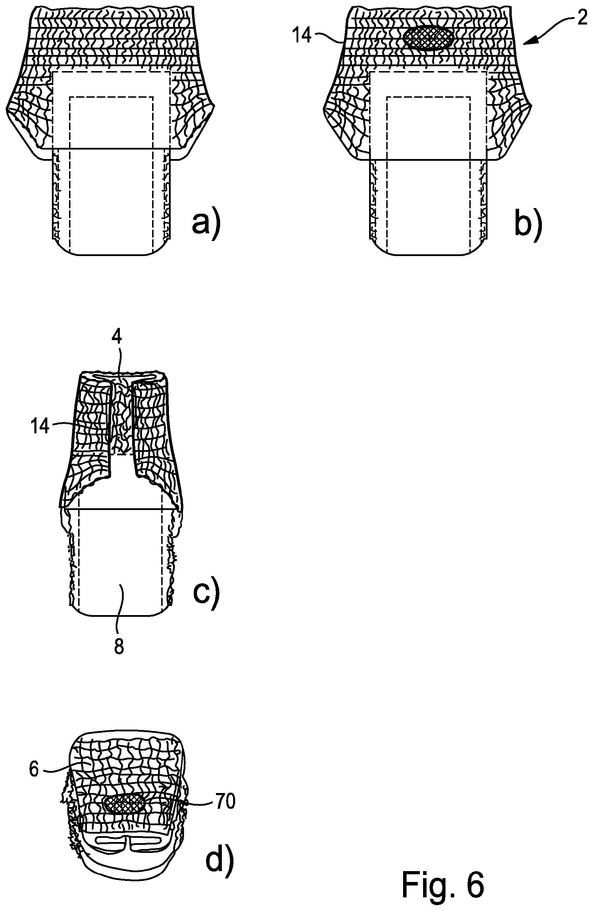

Figur 6 zeigt in verschiedenen Darstellungen a, b, c und d einen Faltvorgang eines Inkontinenzartikels 2, wobei Figur 6a den Inkontinenzartikel 2 von der Vorderseite zeigt und Figur 6b den Inkontinenzartikel 2 von der Rückseite zeigt. Es werden nun zunächst die Seitennahtbereiche 14 beidseits auf den Bauchabschnitt 4 gefaltet und es erfolgt dann eine Faltung um eine Querlinie, so dass der Schrittabschnitt 8 ebenfalls auf dem eingeschlagenen Seitennahtabschnitt 14 und damit auf dem Bauchabschnitt 4 zu liegen kommt, wie es in der abschließenden Darstellung in Figur 6d gezeigt ist. Von der Rückseite her kann daher das erste Kennzeichnungsmittel 70 noch durch die Vlieslagen des Rückenabschnitts 6 hindurchgesehen werden, so dass eine Orientierung und eine Übermittlung der Informationen des Kennzeichnungsmittels 70 nach wie vor auch im gefalteten Zustand möglich ist.

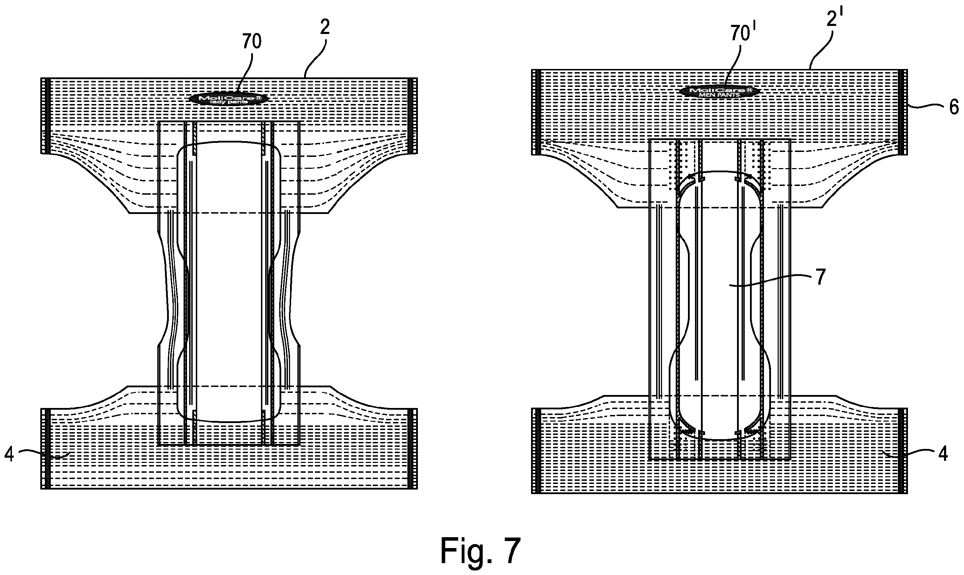

Figur 7 zeigt nun ein ebenfalls durch die Erfindung geschütztes Array umfassend einen ersten Inkontinenzartikel 2 sowie einen zweiten Inkontinenzartikel 2', der sich von dem bisher beschriebenen Inkontinenzartikel 2, der ebenfalls dargestellt ist, insbesondere dadurch unterscheidet, dass der Absorptionskörper 7 beim Inkontinenzartikel 2' mehr in Richtung auf den Bauchabschnitt 4 verschoben ist und darüber hinaus im Bereich der Quermittelachse 30 des Inkontinenzartikels schmaler ausgebildet ist, um so den verschiedenen Bereichen des Flüssigkeitsanfalls besser Rechnung zu tragen. Der erste Inkontinenzartikel 2 bildet dabei mit dem Inkontinenzartikel 2' eine beanspruchte Anordnung ("Array"). Beide Inkontinenzartikel 2 und 2' weisen erste Kennzeichnungsmittel 70 bzw. 70' auf, wobei sich die farbigen Zonen der beiden Inkontinenzartikel 2 und 2' unterscheiden, so dass neben der Positionsinformation, wo der Rückenabschnitt 6 angeordnet ist, eine zusätzliche Information übermittelt wird, die eine Geschlechtsspezifität beinhaltet. Dies kann durch Auswahl einer verschiedenen unterscheidbaren Farbe erfolgen. Darüber hinaus ist es auch möglich, dass das Vliesmaterial der Inkontinenzartikel 2 und 2' im zweiten Bereich um das erste Kennzeichnungsmittel eine voneinander verschiedene Färbung aufweist. Ferner sind die Aussparungen, die das zweite Kennzeichen bilden und vorzugsweise inselartig im Kennzeichnungsmittel 70 bzw. 70' angeordnet sind, so ausgestaltet, dass sie eine zusätzliche weitere Information beinhalten, die ebenfalls auf die Geschlechtsspezifität hindeutet, nämlich im vorliegenden Fall den Hinweis, dass es sich beim ersten Inkontinenzartikel um einen Frauen-Inkontinenzartikel und beim zweiten Inkontinenzartikel um einen Männer-Inkontinenzartikel handelt.

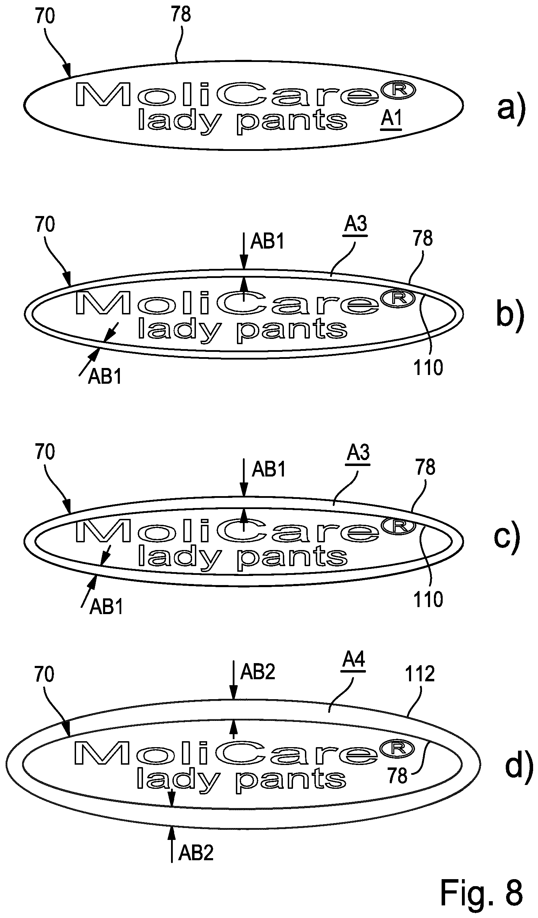

Für die Attraktion des ersten Kennzeichnungsmittels ist eine gewisse flächenhafte Darstellung des ersten Kennzeichens wesentlich, insbesondere betrachtet ausgehend von einer Randumfangslinie 78 des ersten Kennzeichnungsmittels. Ebenso vorteilhaft wird die Attraktion des ersten Kennzeichnungsmittels bzw. damit auch des ersten Kennzeichens durch eine weitgehend solitäre Darstellung erreicht, also in einem äußeren Abstand von der Randumfangslinie 78 weitgehend frei von farbigen Zonen, insbesondere frei von weiteren farbigen Beschichtungen. Dies wird nachfolgend anhand Figuren 8a bis 8d erläutert.

Figuren 8a-8d zeigen das erste Kennzeichnungsmittel 70, wobei in Figur 8a zu illustrativen Zwecken auf die Darstellung unten näher beschriebener imaginärer Abstandslinien verzichtet wurde.

Die Fläche des ersten Kennzeichnungsmittels 70, mithin die durch die Randumfangslinie 78 des ersten Kennzeichnungsmittels 70 einbeschriebene Fläche ist mit A1 bezeichnet. A3 (Figuren 8b und 8c) bezeichnet die Fläche, welche von der Randumfangslinie 78 und einer ersten imaginären Abstandslinie 110 begrenzt wird, wobei die imaginäre Abstandslinie 110 innerhalb der Fläche A1 und in einem konstanten ersten Abstand AB1 von 2 mm nach innen beabstandet von der Randumfangslinie 78 verläuft. Die Größe der Fläche A3, gemessen in mm², beträgt im Falle der Figur 8b 20% der Größe der Fläche A1. In Figur 8c ist die imaginäre Anstandslinie 110 in größerem ersten Abstand AB1 von 3 mm von der Randumfangslinie 78 des ersten Kennzeichnungsmittels 70 skizziert. Die Größe der Fläche A3 beträgt hier 30% der Größe der Fläche A1.

Die Fläche A3 sollte im Wesentlichen durch das erste Kennzeichen gebildet und ausgefüllt sein; der Flächenanteil des zweiten Kennzeichens (also der Aussparungen 76), an der Größe der Fläche A3 beträgt vorteilhaft höchstens 10%, weiter insbesondere höchstens 7%, weiter insbesondere höchstens 5%.

In Figur 8d bezeichnet A4 die Fläche, welche von der Randumfangslinie 78 des ersten Kennzeichnungsmittels 70 und einer zweiten imaginären Abstandslinie 112 begrenzt wird, wobei die zweite imaginäre Abstandslinie 112 außerhalb der Fläche A1 und in einem konstanten zweiten Abstand AB2 von 5 mm beabstandet von der Randumfangslinie 78 verläuft. Die Größe der Fläche A4, gemessen in mm$^2$, beträgt hier in etwa 50% der Größe der Fläche A1.

Die Fläche A4 ist im Wesentlichen, insbesondere zu mindestens 90%, insbesondere zu 100% frei von weiteren farbigen Zonen, insbesondere frei von farbigen Beschichtungen und/oder Bedruckungen.

Figur 9 zeigt nun in einzelnen Schritten eine Bestimmung des Anteils des zweiten Kennzeichens und dessen Verteilung an der Fläche des ersten Kennzeichnungsmittels 70. Hierzu wird das erste Kennzeichnungsmittel 70 (Figur 9a) durch ein Rechteck 100 tangierend umschlossen (Fig. 9b), das bezüglich seiner Kanten in Längs- und Querrichtung 9 bzw. 16 des Inkontinenzartikels 2 verläuft. Mit dem Rechteck 100 werden die jeweils äußersten Punkte des ersten Kennzeichnungsmittels in dessen Quer- und Längsstreckung gerade einbeschrieben. Es erfolgt dann eine Teilung in gleich große Unterrechtecke des Rechtecks 100, die hier mit 101 bezeichnet sind, wobei sowohl in Längs- als auch in Querrichtung 9, 16 jeweils zehn Unterrechtecke 101 vorgesehen sind. Es wird anschließend die Anzahl N1 an Unterrechtecken 101a ermittelt, welche vollständig oder zumindest bereichsweise innerhalb der Randumfangslinie 78 des ersten Kennzeichnungsmittels 70 lokalisiert sind. Es wird dann weiter innerhalb dieser Anzahl N1 der Unterrechtecke 101a die Anzahl N2 derjenigen Unterrechtecke 101b bestimmt (Figur 9c), welche zumindest bereichsweise sowohl das erste Kennzeichen als auch das zweite Kennzeichen aufweisen. Im vorliegenden Fall sind N1 = 88 Unterrechtecke 101a vorhanden, davon sind N2 = 37 Unterrechtecke 101b sowohl mit dem ersten Kennzeichen als auch mit dem zweiten Kennzeichen versehen. Diese 37 Unterrechtecke 101b sind in Figur 9c zur Veranschaulichung dunkel unterlegt. Somit liegt ein Verteilungsgrad von N2/N1*100 von 42 % vor.

Die ersten und zweiten Kennzeichen eines ersten Kennzeichnungsmittels unterscheiden sich vorteilhafterweise auch in deren Farben, gemessen nach CIEL*a*b* und den zueinander ermittelten Farbabständen, dargestellt als ein jeweiliges ΔE*. In Tabelle 1 und 2 sind dazu Ergebnisse für zwei bevorzugte Ausführungsformen von Inkontinenzartikel und dazugehörigem ersten Kennzeichnungsmittel dargestellt.

Tabelle 1: Beispiele für Ausführungsformen mit erstem und zweitem Kennzeichen und deren Farbbestimmung nach CIEL*a*b*

| | | Inkontinenzartikel 1 | | | Inkontinenzartikel 2 | | |
|---|---|---|---|---|---|---|---|
| | | L* | a* | b* | L* | a* | b* |
| Produktaußenseite | Kennzeichen 1, ungedehnt | 76,10 | 15,38 | -4,79 | 50,21 | -3,85 | -3,52 |
| | Kennzeichen 2, ungedehnt | 88,23 | -0,39 | 1,07 | 56,56 | -2,06 | -1,79 |
| | Kennzeichen 1, 200% | 79,71 | 11,27 | -3,37 | 59,06 | -6,04 | -2,44 |
| | Kennzeichen 2, 200% | 90,35 | -0,67 | 2,44 | 66,42 | -2,45 | 0,67 |
| Produktinnenseite | Kennzeichen 1, ungedehnt | 72,78 | 19,98 | -6,45 | 49,32 | -4,86 | -4,12 |
| | Kennzeichen 2, ungedehnt | 87,73 | -0,28 | 1,03 | 55,41 | -2,08 | -1,79 |
| | Kennzeichen 1, 200% | 79,53 | 13,61 | -4,06 | 57,06 | -7,32 | -2,94 |
| | Kennzeichen 2, 200% | 84,26 | -0,45 | 1,82 | 59,42 | -2,00 | -0,70 |

Tabelle 2: Farbabstände von Kennzeichen 1 zu Kennzeichen 2, dargestellt als Delta E und daraus ermittelte Unterschiede

| | Delta E (ΔE*) | | Inkontinenzartikel 1 | Inkontinenzartikel 2 |
|---|---|---|---|---|
| Produktaußenseite | Ungedehnt | ΔE* [a/K1-K2/100%] | 20,74 | 6,82 |
| | 200% | ΔE* [a/K1-K2/200%] | 17,02 | 8,76 |
| Produktinnenseite | Ungedehnt | ΔE* [i/K1-K2/100%] | 26,26 | 7,09 |
| | 200% | ΔE* [i/K1-K2/200%] | 15,96 | 6,24 |
| | | | | |
| Unterschiede/Differenzen | | | | |
| | | | | |
| Produktinnenseite - Produktaußenseite ungedehnt | ΔE* [i/K1-K2/100%] - ΔE* [a/K1-K2/100%] | | 5,52 | 0,28 |
| | | | | |
| Produktinnenseite-Produktaußenseite gedehnt | ΔE* [i/K1-K2/200%] - ΔE* [a/K1-K2/200%] | | -1,06 | -2,52 |
| | | | | |
| innerhalb Produktaußenseite zwischen gedehnt - ungedehnt | ΔE* [a/K1-K2/200%] - ΔE* [a/K1-K2/100%] | | -3,73 | 1,95 |
| | | | | |
| innerhalb Produktinnenseite zwischen gedehnt - ungedehnt | ΔE* [i/K1-K2/200%] - ΔE* [i/K1-K2/100%] | | -10,30 | -0,86 |

In einem Array wie in Figur 7 schematisch dargestellt, unterscheiden sich die ersten und zweiten Kennzeichen voneinander insbesondere in den nach CIEL*a*b* vermessenen Farben und daraus ermittelten Farbabständen, wie exemplarisch in Tabelle 3 dargestellt. Insgesamt bevorzugt sind die Farbabstände, Delta E, im ersten Inkontinenzartikel, für Frauen vorgesehen, größer als im zweiten Inkontinenzartikel, einem für Männer vorgesehenen Artikel.

Tabelle 3: Darstellung eines Arrays mit einem ersten Inkontinenzartikel und einem zweiten Inkontinenzartikel

| | | | Inkontinenzartikel 1 | Inkontinenzartikel 2 | Unterschied Delta E um Faktor |
|---|---|---|---|---|---|
| Produktaußenseite | Ungedehnt | ΔE* [a/K1-K2/100%] | 20,74 | 6,82 | 3,0 |
| | 200% | ΔE* [a/K1-K2/200%] | 17,02 | 8,76 | 1,9 |
| Produktinnenseite | Ungedehnt | ΔE* [i/K1-K2/100%] | 26,26 | 7,09 | 3,7 |
| | 200% | ΔE* [i/K1-K2/200%] | 15,96 | 6,24 | 2,6 |

**Patentansprüche**

1. Inkontinenzartikel (2) für die Aufnahme von Körperausscheidungen mit einem vorderen Bauchabschnitt (4) und einem hinteren Rückenabschnitt (6), die in einer Längsrichtung (9) voneinander beabstandet sind und die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in der Längsrichtung (9) zwischen Bauchabschnitt (4) und Rückenabschnitt (6) erstreckt, wobei der Schrittabschnitt (8), der Bauchabschnitt (4) und der Rückenabschnitt (6) gemeinsam jeweilige Beinöffnungen (19) des Inkontinenzartikels begrenzen, wobei der Bauchabschnitt (4) und der Rückenabschnitt (6) in Quer- oder Hüftumfangsrichtung (16) elastisch dehnbar ausgebildet sind, und wobei der Rückenabschnitt (6) und/oder der Bauchabschnitt (4) ein Vliesmaterial (15) mit zumindest einem ersten Bereich (11) und mit einem zweiten Bereich (13) umfasst, wobei in dem ersten Bereich (11) ein erstes Kennzeichnungsmittel (70) vorgesehen ist, und das erste Kennzeichnungsmittel (70) ein erstes Kennzeichen (72) aufweist, das durch eine flächenhaft erstreckte farbige Zone (73) gebildet ist, und das eine erste Information bereitstellt, wobei die farbige Zone (73) visuell unterscheidbar ist von dem zweiten Bereich (13), **dadurch gekennzeichnet, dass** das erste Kennzeichnungsmittel (70) ein zweites Kennzeichen (74) umfasst, das eine zweite Information bereitstellt, das dadurch gebildet ist, dass in dem ersten Kennzeichen (72) Aussparungen (76) der farbigen Zone (73) vorgesehen sind, wobei das erste Kennzeichnungsmittel (70, 90) innerhalb eines Bereiches zwischen einer am Längsende des Absorptionskörpers, insbesondere am Längsende des Schrittabschnitts, angeordneten und in Querrichtung verlaufenden imaginären Linie und einer die Hüftöffnungskante (18', 18") beschreibenden Linie angeordnet ist.

2. Inkontinenzartikel nach Anspruch 1, **dadurch gekennzeichnet, dass** das erste Kennzeichnungsmittel auf einer körperzugewandten Vlieslage des Vliesmaterials, dabei auf einer körperzugewandten Oberseite oder einer körperabgewandten Oberseite der körperzugewandten Vlieslage aufgebracht ist, oder insbesondere bei einem Vliesmaterial des Bauchabschnitts und/oder Rückenabschnitts umfassend zwei äußere Vlieslagen das erste Kennzeichnungsmittel zwischen den äußeren Vlieslagen, und dabei insbesondere auf der körperabgewandten Oberseite der körperzugewandten Vlieslage aufgebracht ist.

3. Inkontinenzartikel nach einem der vorherigen Ansprüche 1 oder 2, **dadurch gekennzeichnet, dass** ein zweites Kennzeichnungsmittel auf dem das erste Kennzeichnungsmittel aufweisenden Bauch- oder Rückenabschnitt gegenüberliegenden Rücken- oder Bauchabschnitt vorgesehen ist, und dabei insbesondere das zweite Kennzeichnungsmittel auf einer körperabgewandten Vlieslage des Vliesmaterials, dabei auf einer körperzugewandten Oberseite oder einer körperabgewandten Oberseite der körperabgewandten Vlieslage aufgebracht ist, oder insbesondere bei einem Vliesmaterial des Bauchabschnitts und/oder Rückenabschnitts umfassend zwei äußere Vlieslagen das zweite Kennzeichnungsmittel zwischen den äußeren Vlieslagen, und dabei insbesondere auf der körperzugewandten Oberseite der körperabgewandten Vlieslage aufgebracht ist.

4. Inkontinenzartikel nach einem der vorherigen Ansprüche, **dadurch gekennzeichnet, dass** die farbige Zone (73) des ersten Kennzeichnungsmittels eine Bedruckung, insbesondere eine farbige Bedruckung ist.

5. Inkontinenzartikel nach einem der vorherigen Ansprüche, **dadurch gekennzeichnet, dass** das erste und/oder zweite Kennzeichnungsmittel innerhalb eines elastisch dehnbaren Bereiches von Rücken- und/oder Bauchabschnitt angeordnet ist.

6. Inkontinenzartikel nach einem der vorherigen Ansprüche, **dadurch gekennzeichnet, dass** die farbige Zone (73) ausgehend von einer Randumfangslinie (78) des ersten Kennzeichnungsmittels (70) das zweite Kennzeichen derart umschließt, dass die Größe einer Fläche A3, welche von der Randumfangslinie (78) des ersten Kennzeichnungsmittels und einer davon innerhalb und in einem konstanten ersten Abstand AB1 verlaufenden imaginären inneren Abstandslinie (110) begrenzt wird, mindestens 20%, insbesondere mindestens 25%, weiter insbesondere mindestens 30% einer Größe der Fläche A1 beträgt, wobei als die Fläche A1 die durch die Randumfangslinie (78) des ersten Kennzeichnungsmittels (70) eingeschriebene Fläche definiert ist, wobei insbesondere der Flächenanteil des zweiten Kennzeichens an der Fläche A3 höchstens 10%, insbesondere höchstens 7%, weiter insbesondere höchstens 5%, insbesondere höchstens 2% beträgt.

7. Inkontinenzartikel nach einem der vorherigen Ansprüche, **dadurch gekennzeichnet, dass** die Größe einer Fläche A4, welche von einer Randumfangslinie (78) des ersten Kennzeichnungsmittels (70) und einer davon außerhalb und in einem konstanten zweiten Abstand AB2 verlaufenden imaginären Abstandslinie (112) begrenzt wird mindestens 50%, insbesondere mindestens 60%, weiter insbesondere mindestens 70% der Größe der Fläche A1 beträgt, wobei als die Fläche A1 die durch die Randumfangslinie (78) des ersten Kennzeichnungsmittels (70) eingeschriebene Fläche definiert ist, wobei mindestens 90%, insbesondere mindestens 93%, insbesondere mindestens 95% der Fläche A4 frei von weiteren farbigen Zonen, insbesondere als Beschichtungen und/oder Bedruckungen und/oder Änderungen der dem Vliesmaterial zu eigenen optischen visuellen und/oder haptischen Beschaffenheit sind.

8. Inkontinenzartikel nach einem der vorherigen Ansprüche, **dadurch gekennzeichnet, dass** das erste Kennzeichnungsmittel (70) eine durch eine Randumfangslinie (78) eingeschriebene Fläche A1 überfängt, und das zweite Kennzeichen einen Flächenanteil von höchstens 50%, insbesondere höchstens 40% und weiter insbesondere höchstens 30%, weiter insbesondere höchstens 20%, und insbesondere wenigstens 8%, insbesondere wenigstens 10% der vom ersten Kennzeichnungsmittel überfangenen Fläche A1 einnimmt.

9. Inkontinenzartikel nach einem der vorherigen Ansprüche, **dadurch gekennzeichnet, dass** das erste Kennzeichnungsmittel (70) mit einer Randumfangslinie (78) in seinen jeweils äußersten Punkten von einem imaginären Rechteck (100) tangierend eingeschrieben ist, wobei das Rechteck (100) bezüglich seiner Kanten in Längs- und Querrichtung des Inkontinenzartikels angeordnet ist und dabei sowohl in Längsrichtung als auch in Querrichtung in jeweils 10 gleich große Unterrechtecke (101) unterteilt ist, von einer Anzahl N1 an Unterrechtecken (101a), welche vollständig oder zumindest bereichsweise innerhalb der Randumfangslinie (78) lokalisiert sind und davon einer Anzahl N2 derjenigen Unterrechtecken (101b), die zumindest bereichsweise sowohl das erste Kennzeichen als auch das zweite Kennzeichen aufweisen, ein Verteilungsgrad von N2/N1*100% von mindestens 30%, mindestens 35%, insbesondere mindestens 40%, weiter insbesondere mindestens 45% vorgesehen ist.

10. Inkontinenzartikel nach einem der vorherigen Ansprüche, **dadurch gekennzeichnet, dass** das erste Kennzeichnungsmittel (70) in einem planen ausgebreiteten Zustand des Vliesmaterials und des Inkontinenzartikels eine erste Quererstreckung von 10 - 25%, insbesondere von 12 - 23% der maximalen Produktquererstreckung aufweist und/oder das erste Kennzeichnungsmittel (70) eine zweite Quererstreckung gemessen in einem relaxierten, nicht plan ausgebreiteten Zustand des Inkontinenzartikels aufweist, wobei die zweite Quererstreckung 4 - 12%, insbesondere von 5 - 10% bezogen auf die maximale Produktquererstreckung im planen ausgebreiteten Zustand des Vliesmaterials und des Inkontinenzartikels beträgt.

11. Inkontinenzartikel nach einem der vorherigen Ansprüche, **dadurch gekennzeichnet, dass** das erste Kennzeichnungsmittel (70) eine Fläche A1 von 500 - 4000 mm$^2$, insbesondere von 800 - 3700 mm$^2$, weiter insbesondere von 1100 - 3500 mm$^2$ aufweist, dabei die Fläche A1 gemessen in einem planen ausgebreiteten Zustand des Vliesmaterials bzw. des Inkontinenzartikels.

12. Inkontinenzartikel nach einem der vorherigen Ansprüche, **dadurch gekennzeichnet, dass** bei einer Einteilung des Inkontinenzartikels in vier gleichgroße sich in Längsrichtung erstreckende Teilabschnitte, das erste Kennzeichnungsmittel (70) innerhalb der beidseits einer Längsmittelachse (44) des Inkontinenzartikels nächstgelegenen Teilabschnitte, insbesondere im Wesentlichen symmetrisch um die Längsmittelachse (44) angeordnet ist und/oder das erste Kennzeichnungsmittel (70) auf dem Rückenabschnitt (6) angeordnet ist.

13. Inkontinenzartikel nach einem der vorherigen Ansprüche, **dadurch gekennzeichnet, dass** der Schrittabschnitt (8), der Bauchabschnitt (4) und der Rückenabschnitt (6) separate Bauteile sind und der Schrittabschnitt (8) an den Bauchabschnitt (4) und an den Rückenabschnitt (6) in einem jeweiligen Überlappungsbereich (36, 38) unlösbar angefügt ist.

14. Inkontinenzartikel nach einem der vorherigen Ansprüche, **dadurch gekennzeichnet, dass** das erste und/oder zweite Kennzeichnungsmittel (70, 90) außerhalb eines Überlappungsbereichs des Schrittabschnittes mit dem Bauch- und/oder Rückenabschnitts vorgesehen ist und/oder dass das zweite Kennzeichnungsmittel (70, 90) innerhalb eines Bereiches zwischen einer am Längsende des Absorptionskörpers, insbesondere am Längsende des Schrittabschnitts, angeordneten und in Querrichtung verlaufenden imaginären Linie und einer die Hüftöffnungskante (18', 18") beschreibenden Linie angeordnet ist.

15. Inkontinenzartikel nach einem der vorherigen Ansprüche, **dadurch gekennzeichnet, dass** das erste Kennzeichen eine erste Farbe und das zweite Kennzeichen und/oder das Vliesmaterial in einem an das erste Kennzeichen direkt angrenzenden Bereich eine davon unterscheidbare zweite Farbe aufweist, dabei die Farbe jeweils gemessen nach CIEL*a*b* und insbesondere

- a) gemessen jeweils auf einer Produktaußenseite des ersten Kennzeichnungsmittels aufweisenden Bauch- und/ oder Rückenabschnitts und/oder
- b) gemessen jeweils auf einer Produktinnenseite des ersten Kennzeichnungsmittels aufweisenden Bauch- und/ oder Rückenabschnitts,
- wobei die Messungen a) und b) jeweils in einem ungedehnten Zustand des ersten und zweiten Inkontinenzartikels und/oder jeweils in einem auf 200% gedehnten Zustand des Inkontinenzartikels durchgeführt sind und wobei insbesondere ein Farbabstand zwischen der ersten Farbe und der zweiten Farbe, dargestellt als Delta E ($\Delta$E*), 13 bis 30, insbesondere 13 -28 beträgt oder

dass das erste Kennzeichen eine erste Farbe und das zweite Kennzeichen und/oder das Vliesmaterial in einem an das erste Kennzeichen direkt angrenzenden Bereich eine davon unterscheidbare zweite Farbe aufweist, dabei die Farbe jeweils gemessen nach CIEL*a*b*, und insbesondere

- a) gemessen jeweils auf einer Produktaußenseite des ersten Kennzeichnungsmittels aufweisenden Bauch- und/ oder Rückenabschnitts und/oder
- b) gemessen jeweils auf einer Produktinnenseite des ersten Kennzeichnungsmittels aufweisenden Bauch- und/ oder Rückenabschnitts,

wobei die Messungen a) und b) jeweils in einem ungedehnten Zustand des ersten und zweiten Inkontinenzartikels und/oder jeweils in einem auf 200% gedehnten Zustand des Inkontinenzartikels durchgeführt sind, und wobei insbesondere

ein Farbabstand zwischen der ersten Farbe und der zweiten Farbe, dargestellt als Delta E ($\Delta$E*), 5-13, insbesondere 5-10 beträgt.

16. Inkontinenzartikel nach einem der vorherigen Ansprüche, **dadurch gekennzeichnet, dass** zwischen dem ersten Kennzeichen und dem zweiten Kennzeichen und/oder dem Vliesmaterial in einem an das erste Kennzeichen direkt angrenzenden Bereich ein auf einer Produktinnenseite des ersten Kennzeichnungsmittels aufweisenden Bauch- und/oder Rückenabschnitts im ungedehnten Zustand und dabei die Farbe jeweils nach CIEL*a*b* vermessen und ein daraus berechneter Farbabstand, dargestellt als Delta E ($\Delta$E*)[i/K1-K2/100%] vorhanden ist und dass zwischen dem ersten Kennzeichen und dem zweiten Kennzeichen und/oder dem Vliesmaterial in einem an das erste Kennzeichen direkt angrenzenden Bereich, ein auf einer Produktaußenseite des ersten Kennzeichnungsmittels aufweisenden Bauch-und/oder Rückenabschnitts im ungedehnten Zustand dabei die Farbe jeweils nach CIEL*a*b* vermessen und ein daraus berechneter Farbabstand, dargestellt als Delta E ($\Delta$E*)[a/K1-K2/100%] vorhanden ist, wobei Delta E ($\Delta$E*)[i/K1-K2/100%] größer als Delta E ($\Delta$E*)[a/K1-K2/100%] ist, und wobei insbesondere ein Unterschied zwischen Delta E ($\Delta$E*)[i/K1-K2/100%] und Delta E ($\Delta$E*)[a/K1-K2/100%] wenigstens 0,1, insbesondere wenigstens 0,2, insbesondere wenigstens 1, insbesondere wenigstens 2, weiter insbesondere höchstens 10, weiter insbesondere höchstens 9, weiter insbesondere höchstens 8, weiter insbesondere höchstens 7 beträgt und/oder dass zwischen dem ersten Kennzeichen und dem zweiten Kennzeichen und/oder dem Vliesmaterial in einem an das erste Kennzeichen direkt angrenzenden Bereich ein auf einer Produktinnenseite des ersten Kennzeichnungsmittels aufweisenden Bauch-und/ oder Rückenabschnitts im auf 200% gedehnten Zustand und dabei die Farbe jeweils nach CIEL*a*b* vermessen und ein daraus berechneter Farbabstand, dargestellt als Delta E ($\Delta$E*)[i/K1-K2/200%] vorhanden ist und dass zwischen dem ersten Kennzeichen und dem zweiten Kennzeichen und/oder dem Vliesmaterial in einem an das erste Kennzeichen direkt angrenzenden Bereich, ein auf einer Produktaußenseite des ersten Kennzeichnungsmittels aufweisenden Bauch- und/ oder Rückenabschnitts im auf 200% gedehnten Zustand dabei die Farbe jeweils nach CIEL*a*b* vermessen und ein daraus berechneter Farbabstand, dargestellt als Delta E ($\Delta$E*)[a/K1-K2/200%] vorhanden ist, wobei sich ($\Delta$E*)[i/K1-K2/200%] und ($\Delta$E*)[a/K1-K2/200%] um einen Betrag von höchstens 5, weiter insbesondere höchstens 4, weiter insbesondere höchstens 3, insbesondere wenigstens 0,2, insbesondere wenigstens 0,5, insbesondere wenigstens 1 unterscheiden, insbesondere bevorzugt ist dabei der Farbstand $\Delta$E*[i/K1-K2/200%] geringer als der Farbabstand $\Delta$E*[a/K1-K2/200%].

17. Inkontinenzartikel nach einem der vorherigen Ansprüche, **dadurch gekennzeichnet, dass** zwischen dem ersten Kennzeichen und dem zweiten Kennzeichen und/oder dem Vliesmaterial in einem an das erste Kennzeichen direkt angrenzenden Bereich ein auf einer Produktinnenseite des ersten Kennzeichnungsmittels aufweisenden Bauch- und/oder Rückenabschnitts im auf 200% gedehnten Zustand nach CIEL*a*b* ermittelter Farbabstand, dargestellt als Delta E ($\Delta$E*)[i/K1-K2/200%], geringer ist als der nach CIEL*a*b* ermittelte Farbabstand zwischen dem ersten Kennzeichen und dem zweiten Kennzeichen und/oder dem Vliesmaterial in einem an das erste Kennzeichen direkt angrenzenden Bereich auf einer Produktinnenseite des ersten Kennzeichnungsmittels aufweisenden Bauch- und/oder Rückenabschnitts im ungedehnten Zustand, dargestellt als Delta E ($\Delta$E*)[i/K1-K2/100%], insbesondere besteht eine Differenz um einen Betrag von mindestens 5, insbesondere mindestens 6, weiter insbesondere mindestens 8, weiter insbesondere höchstens 14, weiter insbesondere höchstens 13, weiter insbesondere höchstens 12 oder dass zwischen dem ersten Kennzeichen und dem zweiten Kennzeichen und/oder dem Vliesmaterial in einem an das erste Kennzeichen direkt angrenzenden Bereich ein auf einer Produktinnenseite des ersten Kennzeichnungsmittels aufweisenden Bauch- und/oder Rückenabschnitts im auf 200% gedehnten Zustand nach CIEL*a*b* ermittelter Farbabstand, dargestellt als Delta E ($\Delta$E*)[i/K1-K2/200%] geringer ist als der nach CIEL*a*b* ermittelte Farbabstand zwischen dem ersten Kennzeichen und dem zweiten Kennzeichen und/oder dem Vliesmaterial in einem an das erste Kennzeichen direkt angrenzenden Bereich auf einer Produktinnenseite des ersten Kennzeichnungsmittels aufweisenden Bauch-und/oder Rückenabschnitts im ungedehnten Zustand, dargestellt als Delta E ($\Delta$E*) [i/K1-K2/100%], insbesondere besteht eine Differenz um einen Betrag von mindestens 0,2, insbesondere mindestens 0,5, weiter insbesondere höchstens 5, weiter insbesondere höchstens 3.

18. Inkontinenzartikel nach einem der vorherigen Ansprüche, **dadurch gekennzeichnet, dass** zwischen dem ersten Kennzeichen und dem zweiten Kennzeichen und/oder dem Vliesmaterial in einem an das erste Kennzeichen direkt angrenzenden Bereich ein auf einer Produktaußenseite des ersten Kennzeichnungsmittels aufweisenden Bauch-und/oder Rückenabschnitts nach CIEL*a*b* berechnete Farbabstand im auf 200% gedehnten Zustand, dargestellt als Delta E ($\Delta$E*)[a/K1-K2/200%], kleiner ist als der nach CIEL*a*b* berechnete Farbabstand zwischen dem ersten Kennzeichen und dem zweiten Kennzeichen und/oder dem Vliesmaterial in einem an das erste Kennzeichen direkt angrenzenden Bereich auf einer Produktaußenseite des ersten Kennzeichnungsmittels aufweisenden Bauch-und/oder Rückenabschnitts vermessen im ungedehnten Zustand, dargestellt als Delta E ($\Delta$E*)[a/K1-K2/100%], insbesondere besteht eine Differenz um einen Betrag von mindestens 1,5, weiter insbesondere 2, weiter insbesondere mindestens2,5, weiter insbesondere höchstens 7, weiter insbesondere höchstens 6, weiter insbesondere höchstens 5 oder dass zwischen dem ersten Kennzeichen und dem zweiten Kennzeichen und/oder dem Vliesmaterial in einem an das erste Kennzeichen direkt angrenzenden Bereich ein auf einer Produktaußenseite des ersten Kennzeichnungsmittels aufweisenden Bauch-und/oder Rückenabschnitts im auf 200% gedehnten Zustand nach CIEL*a*b* berechnete Farbabstand, dargestellt als Delta E ($\Delta$E*)[a/K1-K2/200%] größer ist als der nach CIEL*a*b* berechnete Farbabstand zwischen dem ersten Kennzeichen und dem zweiten Kennzeichen und/oder dem Vliesmaterial in einem an das erste Kennzeichen direkt angrenzenden Bereich auf einer Produktaußenseite des ersten Kennzeichnungsmittels aufweisenden Bauch- und/oder Rückenabschnitts im ungedehnten Zustand, dargestellt als Delta E ($\Delta$E*) [a/K1-K2/100%], insbesondere besteht eine Differenz um einen Betrag von mindestens 1, weiter insbesondere von mindestens 1,5, weiter insbesondere von höchstens 5, weiter insbesondere von höchstens 4,5 und weiter insbesondere von höchstens 4.

19. Inkontinenzartikel nach einem der vorherigen Ansprüche, **dadurch gekennzeichnet, dass** der Inkontinenzartikel in eine gefaltete Konfiguration verbracht ist, derart dass der das erste Kennzeichnungsmittel (70) aufweisende Bauch- oder Rückenabschnitt (4, 6) in der gefalteten Konfiguration des Inkontinenzartikels näher an einer Außenseite der gefalteten Konfiguration angeordnet ist als der jeweils andere Rücken- oder Bauchabschnitt.

20. Inkontinenzartikel nach einem der vorherigen Ansprüche, **dadurch gekennzeichnet, dass** dem ersten Kennzeichnungsmittel in einem Abstand noch eine Orientierungsmarke (80), vorzugsweise zwischen einer Oberkante des ersten Kennzeichnungsmittels und einer Hüftöffnungskante (18', 18") des Inkontinenzartikels zugeordnet ist, wobei insbesondere die Orientierungsmarke (80) zentriert zu einer Längsmittellinie des ersten Kennzeichnungsmittels angeordnet ist.

21. Inkontinenzartikel nach Anspruch 20, **dadurch gekennzeichnet, dass** die Orientierungsmarke (80) eine dem ersten Kennzeichen identische Farbe aufweist und/oder mit einem identischen die Farbe bildenden Mittel ausgestaltet ist und/oder die Orientierungsmarke (80) zum ersten Kennzeichnungsmittel eine vergleichsweise kleinere Fläche aufweist.

22. Inkontinenzartikel nach einem der vorherigen Ansprüche, **dadurch gekennzeichnet, dass** das zweite Kennzeichnungsmittel (90) im Wesentlichen über die gesamte Produktquerstreckung des Bauch- und/oder Rückenabschnitts angeordnet ist.

23. Anordnung (Array) aus einem ersten Inkontinenzartikel (2) nach einem oder mehreren der vorstehenden Ansprüche und einem zweiten Inkontinenzartikel (2'), für die Aufnahme von Körperausscheidungen mit einem vorderen Bauchabschnitt (4) und einem hinteren Rückenabschnitt (6), die in einer Längsrichtung (9) voneinander beabstandet sind und die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in der Längsrichtung (9) zwischen Bauchabschnitt (4) und Rückenabschnitt (6) erstreckt, wobei der Schrittabschnitt (8), der Bauchabschnitt (4) und der Rückenabschnitt (6) gemeinsam jeweilige Beinöffnungen (19) des Inkontinenzartikels begrenzen, wobei der Bauchabschnitt (4) und der Rückenabschnitt (6) in Quer- oder Hüftumfangsrichtung (16) elastisch dehnbar ausgebildet sind, und wobei der Rückenabschnitt und/oder der Bauchabschnitt ein Vliesmaterial mit zumindest einem ersten Bereich (11) und einem zweiten Bereich (13) umfasst, wobei ein erstes Kennzeichnungsmittel (70') in dem ersten Bereich vorgesehen ist, und das erste Kennzeichnungsmittel ein erstes Kennzeichen aufweist, das durch eine flächenhaft erstreckte farbige Zone (73') gebildet ist, und das eine erste Information bereitstellt, **dadurch gekennzeichnet, dass** das erste Kennzeichnungsmittel (70') des zweiten Inkontinenzartikels (2') ein erstes Kennzeichen umfasst, das eine erste Information bereitstellt, die vom ersten Kennzeichen des ersten Kennzeichnungsmittels (70) des ersten Inkontinenzartikels (2) visuell verschieden ist.

24. Anordnung nach Anspruch 23, **dadurch gekennzeichnet, dass** ein zweites Kennzeichen des zweiten Inkontinenzartikels visuell verschieden ist vom zweiten Kennzeichen des ersten Inkontinenzartikels und insbesondere das zweite Kennzeichen des zweiten Inkontinenzartikels durch insbesondere inselförmige Aussparungen in der farbigen Zone gebildet ist, wobei insbesondere die zweiten Kennzeichen des ersten und zweiten Inkontinenzartikels voneinander verschieden sind.

25. Anordnung nach einem der vorherigen Ansprüche 23 und 24, **dadurch gekennzeichnet, dass** das erste Kennzeichen des ersten und des zweiten Inkontinenzartikel sich unterscheiden in einer Farbe gemessen nach CIEL*a*b*, insbesondere dass ein nach CIEL*a*b* berechneter Farbabstand zwischen den ersten Kennzeichen des ersten und zweiten Inkontinenzartikels, im auf 200% gedehnten Zustand, dargestellt als Delta E ($\Delta$E*)[K1/P1-P2/200%], 24 - 34 beträgt und/oder im ungedehnten Zustand, dargestellt als Delta E ($\Delta$E*)[K1/P1-P2/100%], 29 - 37 beträgt und/oder dass das zweite Kennzeichen des ersten und des zweiten Inkontinenzartikels sich unterscheiden in einer Farbe gemessen nach CIEL*a*b*, insbesondere dass ein nach CIEL*a*b* berechneter Farbabstand zwischen den zweiten Kennzeichen des ersten und zweiten Inkontinenzartikels, im auf 200% gedehnten Zustand - dargestellt als Delta E ($\Delta$E*)[K2/P1-P2/200%], 21-27 und/oder im ungedehnten Zustand, dargestellt als Delta E ($\Delta$E*)[K2/P1-P2/100%], 29 - 35 beträgt.

26. Anordnung (Array) nach einem der vorherigen Ansprüche 23-25, **dadurch gekennzeichnet, dass** der erste Inkontinenzartikel zwischen dem ersten Kennzeichen und dem zweiten Kennzeichen und/oder dem Vliesmaterial in einem an das erste Kennzeichen direkt angrenzenden Bereich einen nach CIEL*a*b* berechneten Farbabstand, dargestellt als ein erstes Delta E ($\Delta$E*)[P1/K1-K2], aufweist und dass der zweite Inkontinenzartikel zwischen dem ersten Kennzeichen und dem zweiten Kennzeichen und/oder dem Vliesmaterial in einem an das erste Kennzeichen direkt angrenzenden Bereich einen nach CIEL*a*b* berechneten Farbabstand, dargestellt als ein zweites Delta E ($\Delta$E*)[P2/K1-K2], aufweist, wobei Delta E ($\Delta$E*)[P1/K1-K2] größer ist als Delta E ($\Delta$E*)[P2/K1-K2], insbesondere mit Delta E ($\Delta$E*)[P1/K1-K2] und Delta E ($\Delta$E*)[P2/K1-K2], dabei die Farbe nach CIEL*a*b*

   - a) gemessen jeweils auf einer Produktaußenseite des ersten Kennzeichnungsmittels aufweisenden Bauch- und/oder Rückenabschnitts; und/oder
   - b) gemessen jeweils auf einer Produktinnenseite des ersten Kennzeichnungsmittels aufweisenden Bauch- und/ oder Rückenabschnitts,

   wobei die Messungen a) und b) jeweils in einem ungedehnten Zustand des ersten und zweiten Inkontinenzartikels und/oder jeweils in einem gedehnten (auf 200%) Zustand des ersten und zweiten Inkontinenzartikels durchgeführt sind und insbesondere Delta E ($\Delta$E*)[P1/K1-K2] größer ist als Delta E ($\Delta$E*)[P2/K1-K2] um einen Faktor von wenigstens 1,2, insbesondere wenigstens 1,5, insbesondere höchstens 5, insbesondere höchstens 4,5; insbesondere höchstens 4.0.

27. Anordnung (Array) nach einem der vorherigen Ansprüche 23-26, **dadurch gekennzeichnet, dass** ein Unterschied der Koordinate L* aus dem CIEL*a*b*-Farbraum zwischen dem ersten Kennzeichen und dem zweiten Kennzeichen und/oder dem Vliesmaterial in einem an das erste Kennzeichen direkt angrenzenden Bereich, dargestellt als Delta L ($\Delta$L*), im ersten Inkontinenzartikel größer ist als im zweiten Inkontinenzartikel, dabei die Farbe nach CIEL*a*b*

   a) gemessen jeweils auf einer Produktaußenseite des ersten Kennzeichnungsmittels aufweisenden Bauch- und/ oder Rückenabschnitts; und/oder
   b) gemessen jeweils auf einer Produktinnenseite des ersten Kennzeichnungsmittels aufweisenden Bauch- und/ oder Rückenabschnitts,

   wobei die Messungen a) und b) jeweils in einem ungedehnten Zustand des ersten und zweiten Inkontinenzartikels und/oder jeweils in einem gedehnten (auf 200%) Zustand des ersten und zweiten Inkontinenzartikels durchgeführt sind.

28. Anordnung (Array) nach einem der vorherigen Ansprüche 23-27, **dadurch gekennzeichnet, dass** der erste Inkontinenzartikel (2) für die Verwendung durch Frauen und/oder als Unisex-Produkt und der zweite Inkontinenzartikel (2') für die Verwendung durch Männer vorgesehen ist, was durch die ersten und/oder zweiten Informationen des ersten und/oder zweiten Kennzeichens gekennzeichnet ist.

29. Anordnung nach einem der vorherigen Ansprüche 23-28, **dadurch gekennzeichnet, dass** das zweite Kennzeichnungsmittel (90) nur auf dem ersten Inkontinenzartikel vorgesehen ist.

**Claims**

1. An incontinence article (2) for the absorption of bodily waste, comprising a front abdominal portion (4) and a rear back portion (6), which are spaced apart from one another in a longitudinal direction (9) and which are connected to one another by the manufacturer at side seam regions (14) on either side to form an abdominal and back belt which is continuous in the transverse direction or circumferential direction (16) of the hip and has a hip opening (18) which is closed in the circumferential direction of the hip, and comprising a crotch portion (8), which has an absorption body (7) and extends in the longitudinal direction (9) between the abdominal portion (4) and the back portion (6), the crotch portion (8), the abdominal portion (4) and the back portion (6) collectively delimiting leg openings (19) of the incontinence article in each case, the abdominal portion (4) and the back portion (6) being designed to be elastically stretchable in the transverse direction or circumferential direction (16) of the hip, and the back portion (6) and/or the abdominal portion (4) comprising a nonwoven material (15) having at least a first region (11) and a second region (13), a first marking means (70) being provided in the first region (11), and the first marking means (70) having a first marking (72) which is formed by an extensive colored zone (73) and which provides a first piece of information, the colored zone (73) being visually distinguishable from the second region (13), **characterized in that** the first marking means (70) comprises a second marking (74) which provides a second piece of information and is formed by cutouts (76) of the colored zone (73) being provided in the first marking (72), the first marking means (70, 90) being arranged within a region between an imaginary line arranged at the longitudinal end of the absorption body, in particular at the longitudinal end of the crotch portion, and extending in the transverse direction, and a line describing the hip opening edge (18', 18").

2. The incontinence article according to claim 1, **characterized in that** the first marking means is applied to a nonwoven layer of the nonwoven material facing the body, in this case to an upper side facing the body or an upper side facing away from the body of the nonwoven layer facing the body, or, in particular in the case of a nonwoven material of the abdominal portion and/or back portion comprising two outer nonwoven layers, **in that** the first marking means is applied between the outer nonwoven layers, and in particular on the upper side facing away from the body of the nonwoven layer facing the body.

3. The incontinence article according to either preceding claim 1 or preceding claim 2, **characterized in that** a second marking means is provided on the back or abdominal portion opposite the abdominal or back portion comprising the first marking means, and in particular the second marking means is applied to a nonwoven layer of the nonwoven material facing away from the body, in this case to an upper side facing the body or an upper side facing away from the body of the nonwoven layer facing away from the body, or, in particular in the case of a nonwoven material of the abdominal portion and/or back portion comprising two outer nonwoven layers, **in that** the second marking means is applied between the outer nonwoven layers, and in particular to the upper side facing the body of the nonwoven layer facing away from the body.

4. The incontinence article according to any one of the preceding claims, **characterized in that** the colored zone (73) of the first marking means is a printed image, in particular a colored printed image.

5. The incontinence article according to any one of the preceding claims, **characterized in that** the first and/or second marking means is arranged within an elastically stretchable region of the back and/or abdominal portion.

6. The incontinence article according to any one of the preceding claims, **characterized in that,** the colored zone (73), starting from a circumferential border line (78) of the first marking means (70), encloses the second marking in such a way that the size of a surface area A3, which is delimited by the circumferential border line (78) of the first marking means and an imaginary inner distance line (110) extending inside thereof and at a constant first distance ABI therefrom, is at least 20%, in particular at least 25%, more particularly at least 30% of the size of the surface area A1, the surface area A1 being defined as the surface area inscribed by the circumferential border line (78) of the first marking means (70), in particular the proportion of the surface area A3 occupied by the second marking being at most 10%, in particular at most 7%, more particularly at most 5%, in particular at most 2%.

7. The incontinence article according to any one of the preceding claims, **characterized in that** the size of a surface area A4 which is delimited by a circumferential border line (78) of the first marking means (70) and an imaginary distance line (112) extending outside thereof and at a constant second distance AB2 is at least 50%, in particular at least 60%, more particularly at least 70% of the size of the surface area A1, the surface area A1 being defined as the surface area inscribed by the circumferential border line (78) of the first marking means (70), at least 90%, in particular at least 93%, in particular at least 95% of the surface area A4 being free of further colored zones, in particular in the form of coatings and/or printed images and/or changes to the nonwoven material's optical visual and/or haptic properties.

8. The incontinence article according to any one of the preceding claims, **characterized in that** the first marking means (70) covers a surface area A1 inscribed by a circumferential border line (78), and the second marking occupies an area proportion of at most 50%, in particular at most 40% and more particularly at most 30%, more particularly at most 20%, and in particular at least 8%, in particular at least 10% of the surface area A1 covered by the first marking means.

9. The incontinence article according to any one of the preceding claims, **characterized in that** the first marking means (70) is tangentially inscribed by a circumferential border line (78) at each of its outermost points of an imaginary rectangle (100), the rectangle (100) being arranged in the longitudinal and transverse directions of the incontinence article with respect to its edges and being divided both longitudinally and transversely into 10 equal-sized sub-rectangles (101) of a number NI of sub-rectangles (101a), either all or regions of which are located within the circumferential border line (78), and of a number N2 of the sub-rectangles (101b), at least regions of which have both the first marking and the second marking, a degree of distribution of N2/N1*100% of at least 30%, at least 35%, in particular at least 40%, more particularly at least 45% is provided.

10. The incontinence article according to any one of the preceding claims, **characterized in that** the first marking means (70) in a flat, unfolded state of the nonwoven material and the incontinence article has a first transverse extension of 10-25%, in particular of 12-23%, of the maximum transverse extension of the product, and/or the first marking means (70) has a second transverse extension measured in a relaxed unfolded state of the incontinence article in which it is not flat, the second transverse extension amounting to 4-12%, in particular 5-10%, of the maximum transverse extension of the product in the flat, unfolded state of the nonwoven material and the incontinence article.

11. The incontinence article according to any one of the preceding claims, **characterized in that** the first marking means (70) has a surface area A1 of 500-4000 mm$^2$, in particular of 800-3700 mm$^2$, more particularly of 1100-3500 mm$^2$, the surface area A1 being measured in a flat, unfolded state of the nonwoven material or of the incontinence article.

12. The incontinence article according to any one of the preceding claims, **characterized in that** when the incontinence article is divided into four equal-sized subportions extending in the longitudinal direction, the first marking means (70) is arranged within the closest subportions on either side of a central longitudinal axis (44) of the incontinence article, in particular substantially symmetrically around the central longitudinal axis (44) and/or the first marking means (70) is arranged on the back portion (6).

13. The incontinence article according to any one of the preceding claims, **characterized in that** the crotch portion (8), the abdominal portion (4) and the back portion (6) are separate components and the crotch portion (8) is inseparably attached to the abdominal portion (4) and to the back portion (6) in an overlapping region (36, 38).

14. The incontinence article according to any one of the preceding claims, **characterized in that** the first and/or second marking means (70, 90) is provided outside of a region of overlap between the crotch portion and the abdominal and/or back portion and/or **in that** the second marking means (70, 90) is arranged within a region between an imaginary line arranged at the longitudinal end of the absorption body, in particular at the longitudinal end of the crotch portion, and running in the transverse direction and a line describing the hip opening edge (18', 18").

15. The incontinence article according to any one of the preceding claims, **characterized in that** the first marking has a first color and the second marking and/or the nonwoven material in a region immediately adjacent to the first marking has a second color that can be distinguished from said first color, the colors each measured according to CIEL*a*b* and in particular

- a) each measured on an abdominal and/or back portion comprising product outside of the first marking means, and/or
- b) each measured on an abdominal and/or back portion comprising product inside of the first marking means,
- the measurements a) and b) each being carried out in an unstretched state of the first and second incontinence article and/or in a state where the incontinence article is stretched to 200% and in particular a color difference between the first color and the second color, represented as Delta E ($\Delta E^*$), is 13 to 30, in particular 13-28 or that the first marking has a first color and the second marking and/or the nonwoven material has a second color that can be distinguished from said first color in a region directly adjacent to the first marking, each of the colors being measured according to CIEL*a*b*, and in particular
- a) each measured on an abdominal and/or back portion comprising product outside of the first marking means, and/or
- b) each measured on an abdominal and/or back portion comprising product inside of the first marking means, the measurements a) and b) each being carried out in an unstretched state of the first and second incontinence article and/or in a state where the incontinence article is stretched to 200%, and in particular a color difference between the first color and the second color, represented as Delta E ($\Delta E^*$), being 5-13, in particular 5-10.

16. The incontinence article according to any one of the preceding claims, **characterized in that** between the first marking and the second marking and/or the nonwoven material in a region immediately adjacent to the first marking, there is an abdominal and/or back portion having on a product inside of the first marking means in the unstretched state, and the color is measured in each case according to CIEL*a*b* and a color difference calculated therefrom, shown as Delta E ($\Delta E^*$)[i/K1-K2/100%] and **in that** between the first marking and the second marking and/or the nonwoven material in a region immediately adjacent to the first marking, there is an abdominal and/or back portion having on a product outside of the first marking means in the unstretched state in this case the color is measured in each case according to CIEL*a*b* and a color difference calculated therefrom, shown as Delta E ($\Delta E^*$)[a/K1-K2/100%], Delta E ($\Delta E^*$)[i/K1-K2/100%] being greater than Delta E ($\Delta E^*$)[a/K1-K2/100%], and in particular a difference between Delta E ($\Delta E^*$)[i/K1-K2/100%] and Delta E ($\Delta E^*$)[a/K1-K2/100%] being at least 0.1, in particular at least 0.2, in particular at least 1, in particular at least 2, more particularly at most 10, more particularly at most 9, more particularly at most 8, more particularly at most 7 and/or in that between the first marking and the second marking and/or the nonwoven material in a region immediately adjacent to the first marking, there is an abdominal and/or back portion having on a product inside of the first marking means in the state stretched to 200% and the color is measured in each case according to CIEL*a*b* and a color difference calculated therefrom, represented as Delta E ($\Delta E^*$)[i/K1-K2/200%], and **in that** between the first marking and the second marking and/or the nonwoven material in a region immediately adjacent to the first marking, there is an abdominal and/or back portion having on a product inside of the first marking means in the state stretched to 200% in this case the color is measured in each case according to CIEL*a*b* and a color difference calculated therefrom, represented as Delta E ($\Delta E^*$)[a/K1-K2/200%], ($\Delta E^*$)[i/K1-K2/200%] and ($\Delta E^*$)[a/K1-K2/200%] differing by an amount of at most 5, more particularly at most 4, more particularly at most 3, in particular at least 0.2, in particular at least 0.5, in particular at least 1, particularly preferably the color level $\Delta E^*$[i/K1-K2/200%] is less than the color difference $\Delta E^*$[a/K1-K2/200%].

17. The incontinence article according to any one of the preceding claims, **characterized in that** between the first marking and the second marking and/or the nonwoven material in a region immediately adjacent to the first marking, a color difference determined according to CIEL*a*b* on an abdominal and/or back portion having on a product inside of the first marking means in the state stretched to 200%, shown as Delta E ($\Delta E^*$)[i/K1-K2/200%], is smaller than the color difference determined according to CIEL*a*b* between the first marking and the second marking and/or the nonwoven material in a region immediately adjacent to the first marking on an abdominal and/or back portion having product inside of the first marking means in the unstretched state, shown as Delta E ($\Delta E^*$)[i/K1-K2/100%], in particular there is a difference by an amount of at least 5, in particular at least 6, more particularly at least 8, more particularly at most 14, more particularly at most 13, more particularly at most 12 or that between the first marking and the second marking and/or the nonwoven material in a region immediately adjacent to the first marking, a color difference determined according to CIEL*a*b* on an abdominal and/or back portion having product inside of the first marking means in the state stretched to 200%, represented as Delta E ($\Delta E^*$)[i/K1-K2/200%], is smaller than the color difference determined according to CIEL*a*b* between the first marking and the second marking and/or the nonwoven material in a region immediately adjacent to the first marking on an abdominal and/or back portion having product inside of the first marking means in the unstretched state, represented as Delta E

($\Delta$E*)[i/K1-K2/100%], in particular there is a difference by an amount of at least 0.2, in particular at least 0.5, more particularly at most 5, more particularly at most 3.

18. The incontinence article according to any one of the preceding claims, **characterized in that** between the first marking and the second marking and/or the nonwoven material in a region immediately adjacent to the first marking, a color difference calculated according to CIEL*a*b* on an abdominal and/or back portion having product outside of the first marking means in the state stretched to 200%, shown as Delta E (AE*)[a/K1-K2/200%], is smaller than the color difference calculated according to CIEL*a*b* between the first marking and the second marking and/or the nonwoven material in a region immediately adjacent to the first marking on an abdominal and/or back portion having product outside of the first marking means measured in the unstretched state, shown as Delta E ($\Delta$E*)[a/K1-K2/100%], in particular there is a difference by an amount of at least 1.5, more particularly 2, more particularly at least 2.5, more particularly at most 7, more particularly at most 6, more particularly at most 5 or that between the first marking and the second marking and/or the nonwoven material in a region immediately adjacent to the first marking, a color difference calculated according to CIEL*a*b* on an abdominal and/or back portion having product outside of the first marking means in the state stretched to 200%, represented as Delta E ($\Delta$E*)[a/K1-K2/200%] is greater than the color difference calculated according to CIEL*a*b* between the first marking and the second marking and/or the nonwoven material in a region immediately adjacent to the first marking on an abdominal and/or back portion having product outside of the first marking means in the unstretched state, represented as Delta E ($\Delta$E*)[a/K1-K2/100%], in particular there is a difference by an amount of at least 1, more particularly at least 1.5, more particularly at most 5, more particularly at most 4.5 and more particularly at most 4.

19. The incontinence article according to any one of the preceding claims, **characterized in that** the incontinence article is placed in a folded configuration such that the abdominal or back portion (4, 6) having the first marking means (70) is closer to the outside of the folded configuration of the incontinence article than the other back or abdominal portion in each case.

20. The incontinence article according to any one of the preceding claims, **characterized in that** an orientation marking (80) is assigned to the first marking means at a spacing, preferably between an upper edge of the first marking means and a hip opening edge (18', 18") of the incontinence article, in particular the orientation marking (80) being centered with respect to a longitudinal center line of the first marking means.

21. The incontinence article according to claim 20, **characterized in that** the orientation marking (80) has a color identical to the first marking and/or is designed having an identical means forming the color and/or the orientation marking (80) has a comparatively smaller surface area than the first marking means.

22. The incontinence article according to any one of the preceding claims, **characterized in that** the second marking means (90) is arranged substantially over the entire product transverse extension of the abdominal and/or back portion.

23. An arrangement (array) consisting of a first incontinence article (2) according to one or more of the preceding claims and a second incontinence article (2'), for the absorption of bodily waste, comprising a front abdominal portion (4) and a rear back portion (6), which are spaced apart from one another in a longitudinal direction (9) and which are connected to one another by the manufacturer at side seam regions (14) on either side to form an abdominal and back belt which is continuous in the transverse direction or circumferential direction (16) of the hip and has a hip opening (18) which is closed in the circumferential direction of the hip, and comprising a crotch portion (8) which has an absorption body (7) and extends in the longitudinal direction (9) between the abdominal portion (4) and the back portion (6), the crotch portion (8), the abdominal portion (4) and the back portion (6) collectively delimiting leg openings (19) of the incontinence article, the abdominal portion (4) and the back portion (6) being elastically stretchable in the transverse direction or circumferential direction (16) of the hip, and the back portion and/or the abdominal portion comprising a nonwoven material having at least a first region (11) and a second region (13), a first marking means (70') being provided in the first region, and the first marking means having a first marking which is formed by an extensive colored zone (73') and provides a first piece of information, **characterized in that** the first marking means (70') of the second incontinence article (2') comprises a first marking which provides a first piece of information that is visually different from the first marking of the first marking means (70) of the first incontinence article (2).

24. The arrangement according to claim 23, **characterized in that** a second marking of the second incontinence article is visually different from the second marking of the first incontinence article and in particular the second marking of the second incontinence article is formed in particular by island-shaped cutouts in the colored zone, in particular the second marking of the first and second incontinence articles are different from one another.

25. The arrangement according to any one of preceding claims 23 and 24, **characterized in that** the first marking of the first and the second incontinence article differ by a color measured according to CIEL*a*b*, in particular **in that** a color difference, which is calculated according to CIEL*a*b*, between the first marking of the first and the second incontinence article, in the state stretched to 200%, represented as Delta E ($\Delta E^*$)[K1/P1-P2/200%], is 24-34 and/or in the unstretched state, represented as Delta E ($\Delta E^*$)[K1/P1-P2/100%], is 29-37 and/or in that the second marking of the first and the second incontinence article differ by a color measured according to CIEL*a*b*, in particular **in that** a color difference calculated according to CIEL*a*b* between the second marking of the first and the second incontinence article, in the state stretched to 200% - represented as Delta E ($\Delta E^*$)[K2/P1-P2/200%], is 21-27 and/or in the unstretched state, represented as Delta E ($\Delta E^*$)[K2/P1-P2/100%], is 29-35.

26. The arrangement (array) according to any one of preceding claims 23-25, **characterized in that** the first incontinence article between the first marking and the second marking and/or the nonwoven material in a region immediately adjacent to the first marking has a color difference calculated according to CIEL*a*b*, represented as a first Delta E ($\Delta E^*$)[P1/K1-K2], and **in that** the second incontinence article has a color difference calculated according to CIEL*a*b* between the first marking and the second marking and/or the nonwoven material in a region immediately adjacent to the first marking, represented as a second Delta E ($\Delta E^*$)[P2/K1-K2], Delta E ($\Delta E^*$)[P1/K1-K2] being greater than Delta E ($\Delta E^*$)[P2/K1-K2], in particular with Delta E ($\Delta E^*$)[P1/K1-K2] and Delta E ($\Delta E^*$)[P2/K1-K2], the color according to CIEL*a*b*

- a) each measured on an abdominal and/or back portion having product outside of the first marking means; and/or
- b) each measured on an abdominal and/or back portion comprising product inside of the first marking means,

the measurements a) and b) each being carried out in an unstretched state of the first and second incontinence article and/or in a stretched (to 200%) state of the first and second incontinence article and in particular Delta E ($\Delta E^*$)[P1/K1-K2] being greater than Delta E ($\Delta E^*$)[P2/K1-K2] by a factor of at least 1.2, in particular at least 1.5, in particular at most 5, in particular at most 4.5; in particular at most 4.0.

27. The arrangement (array) according to any one of preceding claims 23-26, **characterized in that** a difference in terms of the coordinate L* from the CIEL*a*b* color space between the first marking and the second marking and/or the nonwoven material in a region immediately adjacent to the first marking, represented as Delta L ($\Delta L^*$), is greater in the first incontinence article than in the second incontinence article, the color according to CIEL*a*b*

(a) each measured on an abdominal and/or back portion having product outside of the first marking means; and/or
b) each measured on an abdominal and/or back portion having product inside of the first marking means,

the measurements a) and b) each being carried out in an unstretched state of the first and second incontinence articles and/or in a stretched (to 200%) state of the first and second incontinence articles.

28. The arrangement (array) according to any one of preceding claims 23-27, **characterized in that** the first incontinence article (2) is intended for use by women and/or as a unisex product and the second incontinence article (2') is intended for use by men, which is **characterized by** the first and/or second piece of information from the first and/or second marking.

29. The arrangement according to any one of preceding claims 23-28, **characterized in that** the second marking means (90) is provided only on the first incontinence article.

## Revendications

1. Article pour incontinents (2) pour la réception d'excrétions corporelles, avec une section ventrale avant (4) et une section dorsale arrière (6), qui sont espacées l'une de l'autre dans une direction longitudinale (9) et qui sont reliées l'une à l'autre par le fabricant au niveau des régions de couture latérales (14) des deux côtés pour former une bande ventrale et dorsale continue dans la direction transversale ou périphérique de la hanche (16) avec une ouverture de hanche (18) fermée dans la direction périphérique de la hanche, et avec une section d'entrejambe (8) présentant un corps absorbant (7), qui s'étend dans la direction longitudinale (9) entre la section ventrale (4) et la section dorsale (6), dans lequel la section d'entrejambe (8), la section ventrale (4) et la section dorsale (6) délimitent ensemble des ouvertures de jambe (19) respectives de l'article pour incontinents, dans lequel la section ventrale (4) et la section dorsale (6) sont réalisées de manière à pouvoir être étirées élastiquement dans le sens de la circonférence transversale ou de la hanche (16), et dans lequel la section dorsale (6) et/ou la section ventrale (4) comprend un matériau non tissé (15) avec au moins une première région (11) et avec une deuxième région (13), dans lequel un premier moyen d'identification (70) est prévu dans la première région (11), et le premier moyen d'identification (70) présente une première marque distinctive (72), qui est formée par une zone colorée (73) s'étendant en surface et qui fournit une première information, dans lequel la zone colorée (73) peut être distinguée visuellement de la deuxième région (13), **caractérisé en ce que** le premier moyen d'identification (70) comprend une deuxième marque distinctive (74), qui fournit une deuxième information, qui est formée par le fait que des évidements (76) de la zone colorée (73) sont prévus dans la première marque distinctive (72), dans lequel le premier moyen d'identification (70, 90) est disposé à l'intérieur d'une région comprise entre une ligne imaginaire disposée à l'extrémité longitudinale du corps absorbant, en particulier à l'extrémité longitudinale de la section d'entrejambe, et s'étendant dans la direction transversale, et une ligne décrivant le bord d'ouverture de la hanche (18', 18").

2. Article pour incontinents selon la revendication 1, **caractérisé en ce que** le premier moyen d'identification est appliqué sur une couche de non-tissé du matériau non tissé orientée vers le corps, en l'occurrence sur une face supérieure orientée vers le corps ou une face supérieure opposée au corps de la couche de non-tissé orientée vers le corps, ou en particulier dans le cas d'un matériau non tissé de la section ventrale et/ou de la section dorsale comprenant deux couches de non-tissé extérieures, le premier moyen d'identification est appliqué entre les couches de non-tissé extérieures, et ainsi en particulier sur la face supérieure opposée au corps de la couche de non-tissé orientée vers le corps.

3. Article pour incontinents selon l'une quelconque des revendications précédentes 1 ou 2, **caractérisé en ce qu'**un deuxième moyen d'identification est prévu sur la section dorsale ou ventrale en vis-à-vis de la section ventrale ou dorsale présentant le premier moyen d'identification, et en particulier le deuxième moyen d'identification est prévu sur une couche de non-tissé du matériau non tissé orientée vers le corps, ainsi sur une face supérieure orientée vers le corps ou sur une face supérieure opposée au corps de la couche de non-tissé orientée vers le corps, ou en particulier dans le cas d'un matériau non tissé de la section ventrale et/ou de la section dorsale comprenant deux couches de non-tissé extérieures, le deuxième moyen d'identification est appliqué entre les couches de non-tissé extérieures, et en particulier sur la face supérieure orientée vers le corps de la couche de non-tissé opposée au corps.

4. Article pour incontinents selon l'une quelconque des revendications précédentes, **caractérisé en ce que** la zone colorée (73) du premier moyen d'identification est une impression, notamment une impression colorée.

5. Article pour incontinents selon l'une quelconque des revendications précédentes, **caractérisé en ce que** le premier et/ou deuxième moyen d'identification est disposé à l'intérieur d'une région pouvant être étirée élastiquement de la section dorsale et/ou ventrale.

6. Article pour incontinents selon l'une quelconque des revendications précédentes, **caractérisé en ce que** la zone colorée (73) entoure la deuxième marque distinctive à partir d'une ligne périphérique marginale (78) de la deuxième marque distinctive (70), de telle sorte que la taille d'une surface A3, laquelle est délimitée par la ligne périphérique marginale (78) de la première marque distinctive et une ligne d'écartement intérieure imaginaire (110) s'étendant à l'intérieur de celle-ci et à une première distance constante AB1, au moins 20 %, en particulier au moins 25 %, plus particulièrement au moins 30 % d'une taille de la surface A1, dans lequel la surface A1 est définie comme la surface inscrite par la ligne périphérique marginale (78) du premier moyen d'identification (70), dans lequel la part de surface de la deuxième marque distinctive sur la surface A3 est au plus de 10 %, en particulier au plus de 7 %, plus particulièrement au plus de 5 %, en particulier au plus de 2 %.

7. Article pour incontinents selon l'une quelconque des revendications précédentes, **caractérisé en ce que** la taille d'une surface A4, laquelle est délimitée par une ligne périphérique marginale (78) du premier moyen d'identification (70) et une ligne d'écartement imaginaire (112) s'étendant à l'extérieur de celle-ci et à une deuxième distance constante AB2, est d'au moins 50 %, en particulier d'au moins 60 %, plus particulièrement d'au moins 70 % de la taille de la surface A1, dans lequel la surface inscrite par la ligne périphérique marginale (78) du premier moyen d'identification (70) est définie comme la surface A1, dans lequel au moins 90 %, en particulier au moins 93 %, en particulier au moins 95 % de la surface A4 sont exempts d'autres zones colorées, en particulier sous forme de revêtements et/ou d'impressions et/ou de modifications de la nature visuelle et/ou captique propre au matériau non tissé.

8. Article pour incontinents selon l'une quelconque des revendications précédentes, **caractérisé en ce que** le premier moyen d'identification (70) recouvre une surface A1 inscrite par une ligne périphérique marginale (78), et le deuxième moyen d'identification occupe une part de surface d'au plus 50 %, en particulier d'au plus 40 %, et plus particulièrement d'au plus 30 %, plus particulièrement d'au plus 20 %, et en particulier d'au moins 8 %, en particulier d'au moins 10 % de la surface A1 recouverte par le premier moyen d'identification.

9. Article pour incontinents selon l'une quelconque des revendications précédentes, **caractérisé en ce que** le premier moyen d'identification (70) est inscrit avec une ligne périphérique marginale (78) dans ses points respectivement les plus extérieurs en étant tangent à un rectangle (100) imaginaire, dans lequel le rectangle (100) est disposé par rapport à ses bords dans la direction longitudinale et transversale de l'article pour incontinents et est ainsi divisé aussi bien dans la direction longitudinale que dans la direction transversale en respectivement 10 sous-rectangles (101) de même taille, d'un nombre N1 de sous-rectangles (101a), lesquels sont localisés entièrement ou au moins par endroits à l'intérieur de la ligne périphérique marginale (78), et d'un nombre N2 de sous-rectangles (101b), qui présentent au moins par endroits aussi bien la première marque distinctive que la deuxième marque distinctive, un degré de répartition de N2/N1*100 % d'au moins 30 %, d'au moins 35 %, en particulier d'au moins 40 %, plus particulièrement d'au moins 45 % est prévu.

10. Article pour incontinents selon l'une quelconque des revendications précédentes, **caractérisé en ce que** le premier moyen d'identification (70) présente une première extension transversale de 10 à 25 %, en particulier de 12 à 23 % de l'extension transversale maximale du produit, dans un état déployé plan du matériau non tissé et de l'article pour incontinents, et/ou le premier moyen d'identification (70) présente une deuxième extension transversale mesurée dans un état déployé détendu, non plan de l'article pour incontinents, dans lequel la deuxième extension transversale est de 4 à 12 %, en particulier de 5 à 10 % par rapport à l'extension transversale maximale du produit dans l'état déployé plan du matériau non tissé et de l'article pour incontinents.

11. Article pour incontinents selon l'une quelconque des revendications précédentes, **caractérisé en ce que** le premier moyen d'identification (70) présente une surface A1 de 500 à 4000 mm$^2$, en particulier de 800 à 3700 mm$^2$, plus particulièrement de 1100 à 3500 mm$^2$, ainsi la surface A1 est mesurée dans un état déployé plan du matériau non tissé ou de l'article pour incontinents.

12. Article pour incontinents selon l'une quelconque des revendications précédentes, **caractérisé en ce que**, lors d'une division de l'article pour incontinents en quatre sections partielles de même taille s'étendant dans la direction longitudinale, le premier moyen d'identification (70) est disposé à l'intérieur des sections partielles les plus proches de part et d'autre d'un axe médian longitudinal (44) de l'article pour incontinents, en particulier de manière sensiblement symétrique autour de l'axe médian longitudinal (44) et/ou le premier moyen d'identification (70) est disposé sur la section dorsale (6).

13. Article pour incontinents selon l'une quelconque des revendications précédentes, **caractérisé en ce que** l'étape d'entrejambe (8), la section ventrale (4) et la section dorsale (6) sont des composants séparés, et la section d'entrejambe (8) est attachée de manière non détachable à la section ventrale (4) et à la section dorsale (6) dans une région de chevauchement (36, 38) respective.

14. Article pour incontinents selon l'une quelconque des revendications précédentes, **caractérisé en ce que** le premier et/ou deuxième moyen d'identification (70, 90) est prévu à l'extérieur d'une région de chevauchement de la section d'entrejambe avec la section ventrale et/ou dorsale et/ou **en ce que** le deuxième moyen d'identification (70, 90) est disposé à l'intérieur d'une région entre une ligne imaginaire disposée à l'extrémité longitudinale du corps absorbant, en particulier à l'extrémité longitudinale de la section d'entrejambe, et s'étendant dans la direction transversale, et une ligne décrivant le bord d'ouverture de la hanche (18', 18").

15. Article pour incontinents selon l'une quelconque des revendications précédentes, **caractérisé en ce que** la première marque distinctive présente une première couleur et la deuxième marque distinctive et/ou le matériau non tissé présente, dans une région directement adjacente à la première marque distinctive, une deuxième couleur pouvant être distinguée de celle-ci, la couleur étant mesurée respectivement selon CIEL*a*b* et en particulier

- a) mesurée respectivement sur une face extérieure de produit de la section ventrale et/ou dorsale présentant le premier moyen d'identification et/ou
- b) mesurée respectivement sur une face intérieure de produit de la section ventrale et/ou dorsale présentant le premier moyen d'identification,

- dans lequel les mesures a) et b) sont mises en oeuvre respectivement dans un état non étiré du premier et deuxième article pour incontinents et/ou respectivement dans un état étiré à 200 % de l'article pour incontinents, et dans lequel en particulier un écart de couleur entre la première couleur et la deuxième couleur, représenté comme delta E ($\Delta$E*), est de 13 à 30, en particulier de 13 à 28, ou que la première marque distinctive présente une première couleur et la deuxième marque distinctive et/ou le matériau non tissé dans une région directement adjacente à la première marque distinctive une deuxième couleur qui peut en être distinguée, la couleur étant respectivement mesurée selon CIEL*a*b*, et en particulier

- a) mesurée respectivement sur une face extérieure de produit de la section ventrale et/ou dorsale présentant le premier moyen d'identification et/ou

- b) mesurée respectivement sur une face intérieure de produit de la section ventrale et/ou dorsale présentant le premier moyen d'identification,

dans lequel les mesures a) et b) sont mises en oeuvre respectivement dans un état non étiré du premier et deuxième article pour incontinents et/ou respectivement dans un état étiré à 200 % de l'article pour incontinents, et dans lequel en particulier

un écart de couleur entre la première couleur et la deuxième couleur, représenté comme delta E ($\Delta$E*), est de 5 à 13, en particulier de 5 à 10.

16. Article pour incontinents selon l'une quelconque des revendications précédentes, **caractérisé en ce que**, entre la première marque distinctive et la deuxième marque distinctive et/ou le matériau non tissé, dans une région directement adjacente à la première marque distinctive, un sur une face intérieure de produit de la section ventrale et/ou dorsale présentant le premier moyen d'identification dans l'état non étiré et ainsi la couleur est mesurée respectivement selon CIEL*a*b* et un écart de couleur calculé à partir de cela, représenté comme delta E ($\Delta$E*)[1/K1-K2/100 %] est présent et qu'entre la première marque distinctive et la deuxième marque distinctive et/ou le matériau non tissé dans une région directement adjacente à la première marque distinctive, un sur une face extérieure de produit de la section ventrale et/ou dorsale présentant le premier moyen d'identification dans l'état non étiré, la couleur est ainsi mesurée respectivement selon CIEL*a*b* et un écart de couleur calculé à partir de cela, représenté comme delta E ($\Delta$E*)[a/K1-K2/100 %] est présent, dans lequel delta E ($\Delta$E*)[1/K1-K2/100 %] est supérieur à delta E ($\Delta$E*)[a/K1-K2/100 %], et dans lequel en particulier une différence entre delta E ($\Delta$E*)[i/K1-K2/100 %] et delta E ($\Delta$E*)[a/K1-K2/100 %] est d'au moins 0,1, en particulier d'au moins 0,2, en particulier d'au moins 1, en particulier d'au moins 2, plus particulièrement d'au plus 10, plus particulièrement d'au plus 9, plus particulièrement d'au plus 8, plus particulièrement d'au plus 7 et/ou qu'entre la première marque distinctive et la deuxième marque distinctive et/ou le matériau non tissé dans une région directement adjacente à la première marque distinctive, un sur une face intérieure de produit de la section ventrale et/ou dorsale présentant le premier moyen d'identification dans l'état étiré à 200 % et la couleur est ainsi mesurée respectivement selon CIEL*a*b* et un écart de couleur calculé à partir de cela, représenté comme delta E ($\Delta$E*)[i/K1-K2/200 %] est présent et qu'entre la première marque distinctive et la deuxième marque distinctive et/ou le matériau non tissé dans une région directement adjacente à la première marque distinctive, un sur une face extérieure de produit de la section ventrale et/ou dorsale présentant le premier moyen d'identification dans l'état étiré à 200 %, la couleur est ainsi mesurée respectivement selon CIEL*a*b* et un écart de couleur calculé à partir de cela, représenté comme delta E ($\Delta$E*)[a/K1-K2/200 %] est présent, dans lequel ($\Delta$E*)[i/K1-K2/200 %] et ($\Delta$E*)[a/K1-K2/200 %] se distinguent d'une valeur d'au plus 5, plus particulièrement d'au plus 4, plus particulièrement d'au plus 3, en particulier d'au moins 0,2, en particulier d'au moins 0,5, en particulier d'au moins 1, de manière particulièrement préférée l'écart de couleur DE*[i/K1-K2/200 %] est ainsi inférieur à l'écart de couleur DE*[a/K1-K2/200 %].

17. Article pour incontinents selon l'une quelconque des revendications précédentes, **caractérisé en ce que**, entre la première marque distinctive et la deuxième marque distinctive et/ou le matériau non tissé dans une région directement adjacente à la première marque distinctive, un écart de couleur déterminé selon CIEL*a*b* dans l'état étiré à 200 % sur une face intérieure de produit de la section ventrale et/ou dorsale présentant le premier moyen d'identification, représenté comme delta E ($\Delta$E*)[i/K1-K2/200 %], est inférieur à l'écart de couleur déterminé selon CIEL*a*b* entre la première marque distinctive et la deuxième marque distinctive et/ou le matériau non tissé dans une région directement adjacente à la première marque distinctive sur une face intérieure de produit de la section ventrale et/ou dorsale présentant le premier moyen d'identification dans l'état non étiré, représenté comme delta E ($\Delta$E*)[i/K1-K2/100 %], en particulier il existe une différence d'une valeur d'au moins 5, en particulier d'au moins 6, plus particulièrement d'au moins 8, plus particulièrement d'au plus 14, plus particulièrement d'au plus 13, plus particulièrement d'au plus 12 ou qu'entre la première marque distinctive et la deuxième marque distinctive et/ou le matériau non tissé dans une région directement adjacente à la première marque distinctive, un écart de couleur déterminé selon CIEL*a*b* dans l'état étiré à 200 % sur une face intérieure de produit d'une section ventrale et/ou dorsale présentant le premier moyen d'identification, représenté comme delta E ($\Delta$E*)[i/K1-K2/200 %] est inférieur à l'écart de couleur déterminé selon CIEL*a*b* dans l'état non étiré entre la première marque distinctive et la deuxième marque distinctive et/ou le matériau non tissé dans une région directement adjacente à la première marque distinctive sur une face intérieure de produit de la section ventrale et/ou dorsale présentant le premier moyen d'identification, représenté par delta E (ΔE*)[i/K1-K2/100 %], en particulier il existe une différence d'une valeur d'au moins 0,2, en particulier d'au moins 0,5, plus particulièrement d'au plus 5, plus particulièrement d'au plus 3.

18. Article pour incontinents selon l'une quelconque des revendications précédentes, **caractérisé en ce que**, entre la première marque distinctive et la deuxième marque distinctive et/ou le matériau non tissé dans une région directement adjacente à la première marque distinctive, un écart de couleur calculé selon CIEL*a*b* dans l'état étiré à 200 % sur une face extérieure de produit de la section ventrale et/ou dorsale présentant le premier article d'identification, représenté comme delta E (ΔE*)[a/K1-K2/200 %], est inférieur à l'écart de couleur calculé selon CIEL*a*b* entre la première marque distinctive et la deuxième marque distinctive et/ou le matériau non tissé dans une région directement adjacente à la première marque distinctive sur une face extérieure de produit de la première section ventrale et/ou dorsale présentant le premier moyen d'identification, mesuré dans l'état non étiré, représenté comme delta E (ΔE*)[a/K1-K2/100 %], en particulier il existe une différence d'une valeur d'au moins 1,5, plus particulièrement de 2, plus particulièrement d'au moins 2,5, plus particulièrement d'au plus 7, plus particulièrement d'au plus 6, plus particulièrement d'au plus 5, ou qu'entre la première marque distinctive et la deuxième marque distinctive et/ou le matériau non tissé dans une région directement adjacente à la première marque distinctive, un écart de couleur calculé selon CIEL*a*b* sur une face extérieure de produit de la section ventrale et/ou dorsale présentant le premier moyen d'identification dans l'état étiré à 200 %, représenté comme delta E (ΔE*)[a/K1-K2/200 %] est supérieur à l'écart de couleur calculé selon CIEL*a*b* entre la première marque distinctive et la deuxième marque distinctive et/ou le matériau non tissé dans une région directement adjacente à la première marque distinctive sur une une face extérieure de produit de la section ventrale et/ou dorsale présentant le premier moyen d'identification dans l'état non étiré, représenté comme delta E (ΔE*)[a/K1-K2/100 %], en particulier il existe une différence d'une valeur d'au moins 1, plus particulièrement d'au moins 1,5, plus particulièrement d'au plus 5, plus particulièrement d'au plus 4,5 et plus particulièrement d'au plus 4.

19. Article pour incontinents selon l'une quelconque des revendications précédentes, **caractérisé en ce que** l'article pour incontinents est placé dans une configuration pliée, de telle sorte que, dans la configuration pliée de l'article pour incontinents, la section ventrale ou dorsale (4, 6) présentant le premier moyen d'identification (70) est disposée plus près d'une face extérieure de la configuration pliée que l'autre section dorsale ou ventrale.

20. Article pour incontinents selon l'une quelconque des revendications précédentes, **caractérisé en ce qu'**un repère d'orientation (80), de préférence entre un bord supérieur du premier moyen d'identification et un bord d'ouverture de hanche (18', 18'') de l'article pour incontinents, est encore associé au premier moyen d'identification à une certaine distance, dans lequel le repère d'orientation (80) est en particulier disposé de manière centrée par rapport à une ligne médiane longitudinale du premier moyen d'identification.

21. Article pour incontinents selon la revendication 20, **caractérisé en ce que** le repère d'orientation (80) présente une couleur identique à la première marque distinctive et/ou est réalisé avec un moyen identique formant la couleur et/ou le repère d'orientation (80) présente une surface comparativement plus petite par rapport au premier moyen d'identification.

22. Article pour incontinents selon l'une quelconque des revendications précédentes, **caractérisé en ce que** le deuxième moyen d'identification (90) est disposé sensiblement sur toute l'étendue transversale de produit de la section ventrale et/ou dorsale.

23. Ensemble composé d'un premier article pour incontinents (2) selon une ou plusieurs des revendications précédentes et d'un deuxième article pour incontinents (2') pour la réception d'excrétions corporelles, avec une section ventrale avant (4) et une section dorsale arrière (6), qui sont espacées l'une de l'autre dans une direction longitudinale (9) et qui sont reliées l'une à l'autre par le fabricant au niveau des régions de couture latérales (14) des deux côtés pour former une bande ventrale et dorsale continue dans la direction transversale ou périphérique de la hanche (16) avec une ouverture de hanche (18) fermée dans la direction périphérique de la hanche, et avec une section d'entrejambe (8) présentant un corps absorbant (7), qui s'étend dans la direction longitudinale (9) entre la section ventrale (4) et la section dorsale (6), dans lequel la section d'entrejambe (8), la section ventrale (4) et la section dorsale (6) délimitent ensemble des ouvertures de jambe (19) respectives de l'article pour incontinents, dans lequel la section ventrale (4) et la section dorsale (6) sont réalisées de manière à pouvoir être étirées élastiquement dans la direction périphérique transversale ou de la hanche (16), et dans lequel la section dorsale et/ou la section ventrale comprend un matériau non tissé avec au moins une première région (11) et une deuxième région (13), dans lequel un premier moyen d'identification (70') est prévu dans la première région, et le premier moyen d'identification présente une première marque distinctive, qui est formée par une zone colorée s'étendant en surface (73') et qui fournit une première information, **caractérisé en ce que** le premier moyen d'identification (70') du deuxième article pour incontinents (2') comprend une première marque distinctive, qui fournit une première information qui est visuellement différente de la première marque distinctive du premier moyen d'identification (70) du premier article pour incontinents (2).

24. Dispositif selon la revendication 23, **caractérisé en ce qu'**une deuxième marque distinctive du deuxième article pour incontinents est visuellement différente de la deuxième marque distinctive du premier article pour incontinents et en particulier la deuxième marque distinctive du deuxième article pour incontinents est formée par des évidements en particulier en forme d'îlots dans la zone colorée, dans lequel en particulier les deuxièmes marque distinctives du premier et deuxième article pour incontinents sont en particulier différentes l'une de l'autre.

25. Ensemble selon l'une quelconque des revendications 23 et 24 précédentes, **caractérisé en ce que** la première marque distinctive du premier et du deuxième article pour incontinents se distinguent par une couleur mesurée selon CIEL*a*b*, en particulier qu'un écart de couleur calculé selon CIEL*a*b* entre les premières marque distinctives du premier et deuxième article pour incontinents, dans l'état étiré à 200 %, représenté comme delta E ($\Delta$E*) [K1/P1-P2/200 %], est de 24 à 34 et/ou dans l'état non étiré, représenté comme delta E ($\Delta$E*) [K1/P1-P2/100 %], 29 à 37 et/ou que la deuxième marque distinctive du premier et du deuxième article pour incontinents se distinguent par une couleur mesurée selon CIEL*a*b*, en particulier qu'un écart de couleur calculé selon CIEL*a*b* entre les deux marque distinctives du premier et deuxième article pour incontinents, dans l'état étiré à 200 % - représenté comme delta E ($\Delta$E*) [K2/P1-P2/200 %], est de 21 à 27 et/ou dans l'état non étiré, représenté comme delta E ($\Delta$E*) [K2/P1-P2/100 %], est de 29 à 35.

26. Ensemble selon l'une quelconque des revendications précédentes 23-25, **caractérisé en ce que** le premier article pour incontinents présente, entre la première marque distinctive et la deuxième marque distinctive et/ou le matériau non tissé, dans une région directement adjacente à la première marque distinctive, un écart de couleur calculé selon CIEL*a*b*, représenté par un premier delta E ($\Delta$E*) [P1/K1-K2], et **en ce que** le deuxième article pour incontinents présente, entre la première marque distinctive et la deuxième marque distinctive et/ou le matériau non tissé, dans une région directement adjacente à la première marque distinctive, un écart de couleur calculé selon CIEL*a*b*, représenté par un deuxième delta E ($\Delta$E*) [P2/K1-K2], dans lequel delta E ($\Delta$E*) [P1/K1-K2] est supérieur à delta E ($\Delta$E*) [P2/K1-K2], en particulier avec delta E ($\Delta$E*) [P1/K1-K2] et delta E ($\Delta$E*)[P2/K1-K2], la couleur étant selon CIEL*a*b*

   - a) mesurée respectivement sur une face extérieure de produit de la section ventrale et/ou dorsale présentant le premier moyen d'identification ; et/ou
   - b) mesurée respectivement sur une face intérieure de produit de la section ventrale et/ou dorsale présentant le premier moyen d'identification,

   dans lequel les mesures a) et b) sont mises en oeuvre respectivement dans un état non étiré du premier et deuxième article pour incontinents et/ou respectivement dans un état étiré (à 200 %) du premier et deuxième article pour incontinents, et en particulier delta E ($\Delta$E*)[P1/K1-K2] est supérieur à delta E ($\Delta$E*)[P2/K1-K2] d'un facteur d'au moins 1,2, en particulier d'au moins 1,5, en particulier d'au plus 5, en particulier d'au plus 4,5 ; en particulier d'au plus 4,0.

27. Ensemble selon l'une quelconque des revendications précédentes 23-26, **caractérisé en ce qu'**une différence de coordonnées L* de l'espace colorimétrique CIEL*a*b* entre la première marque distinctive et la deuxième marque distinctive et/ou le matériau non tissé dans une région directement adjacente à la première marque distinctive, représentée comme delta L (AL*), est plus grande dans le premier article pour incontinents que dans le deuxième article pour incontinents, la couleur étant selon CIEL*a*b*

   a) mesurée respectivement sur une face extérieure de produit de la section ventrale et/ou dorsale présentant le premier moyen d'identification ; et/ou
   b) mesurée respectivement sur une face intérieure de produit de la section ventrale et/ou dorsale présentant le premier moyen d'identification,

   dans lequel les mesures a) et b) sont mises en oeuvre respectivement dans un état non étiré du premier et deuxième article pour incontinents et/ou respectivement dans un état étiré (à 200 %) du premier et deuxième article pour incontinents.

28. Ensemble selon l'une quelconque des revendications précédentes 23-27, **caractérisé en ce que** le premier article pour incontinents (2) est destiné à être utilisé par des femmes et/ou comme produit unisexe et le deuxième article pour incontinents (2') est destiné à être utilisé par des hommes, ce qui est **caractérisé par** les premières et/ou deuxièmes informations de la première et/ou deuxième marque distinctive.

29. Ensemble selon l'une quelconque des revendications précédentes 23 à 28, **caractérisé en ce que** le deuxième moyen d'identification (90) est prévu uniquement sur le premier article pour incontinents.

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2841038 A1
- EP 1815831 A1
- US 2007265591 A
- US 2008108967 A